# EUROPÄISCHE PATENTSCHRIFT

(11) **EP 4 316 385 B1**
(45) Veröffentlichungstag und Bekanntmachung des Hinweises auf die Patenterteilung: **24.07.2024**
(21) Anmeldenummer: 22188980.1
(22) Anmeldetag: 05.08.2022
(51) Int. Cl.: A61B 10/02, A61B 17/34, A61B 10/04, A61B 90/00, A61B 17/00

(54) **VORRICHTUNG ZUM FESTLEGEN EINER EINDRINGTIEFE EINES ROHR- ODER STANGENFÖRMIGEN SCHIEBETEILS IN EINEM AUFNAHMETEIL UND MEDIZINISCHES HANDSTÜCK MIT SOLCHEN VORRICHTUNGEN**
DEVICE FOR FIXING A PENETRATION DEPTH OF A TUBULAR OR BAR-SHAPED SLIDING PART IN A RECEIVING PART AND MEDICAL HANDPIECE WITH SUCH DEVICES
DISPOSITIF PERMETTANT DE DÉTERMINER UNE PROFONDEUR D'INSERTION D'UNE PARTIE COULISSANTE EN FORME DE TIGE OU DE TUBE DANS UNE PARTIE RÉCEPTRICE ET PIÈCE À MAIN MÉDICALE DOTÉE DES TELS DISPOSITIFS

(43) Veröffentlichungstag der Anmeldung: 07.02.2024
(73) Patentinhaber: Medi-Globe GmbH, 83101 Rohrdorf-Achenmühle (DE)
(72) Erfinder: STEINIGEWEG, Martin, 83071 Stephanskirchen (DE)
(74) Vertreter: Hofstetter, Schurack & Partner

(56) Entgegenhaltungen:
- EP-B1- 2 531 122
- CN-B- 105 943 092
- US-A- 5 941 853
- US-A1- 2006 009 677
- US-A1- 2018 317 750

## Beschreibung

Die Erfindung betrifft eine Vorrichtung zum Festlegen einer Eindringtiefe eines rohr- oder stangenförmigen Schiebeteils, insbesondere eines medizinischen Aspirationsnadelträger-Teils, in einem Aufnahmeteil. Das Schiebeteil ist somit entlang der Längsachse in dem Aufnahmeteil verstellbar oder bewegbar, insbesondere z.B. verschiebbar. Die Erfindung betrifft auch ein medizinisches Handstück für ein medizinisches Gerät, mit einer ersten und zweiten Vorrichtung der vorgenannten Art. Das Handstück kann zum Beispiel von dem medizinischen Gerät umfasst sein, und zum Beispiel für endoskopische Arbeiten oder Eingriffe in der Chirurgie verwendet wird.

Eine Vorrichtung und ein medizinisches Handstück der vorgenannten Art sind aus der EP 2 531 122 B1 bekannt. Die dort beschriebene Vorrichtung wird unter der Bezeichnung "Twistlock" geführt. Die Vorrichtung nutzt eine Klemmeinrichtung, um das Schiebeteil relativ zu dem Aufnahmeteil an einer gewünschten Position entlang der Längsachse zu positionieren und dort zu blockieren.

Die beschriebene Vorrichtung umfasst ein Griffstück, welches das Schiebeteil umgibt. Das Griffstück ist relativ zu dem Schiebeteil und/oder dem Aufnahmeteil zum Verschieben entlang einer Längsachse des Schiebeteils ausgebildet. Das heißt, das Griffstück und das Schiebeteil bzw. das Aufnahmeteil können translatorisch zueinander bewegt werden. Um das Verschieben zu ermöglichen, können das Aufnahmeteil und das Griffstück eine Aufnahme oder Öffnung umfassen, durch die das Schiebeteil aufgenommen ist und geschoben werden kann.

Das Griffstück kann zum Beispiel an dem Aufnahmeteil angebracht oder befestigt sein. Somit kann das Aufnahmeteil gemeinsam mit dem Griffstück beim Verschieben des Griffstücks entlang der Längsachse relativ zu dem Schiebeteil verschoben werden. Alternativ kann das Griffstück als separates Element zu dem Aufnahmeteil ausgebildet sein. Beim Verschieben entlang der Längsachse des Schiebeteils kann das Griffstück somit auch relativ zu dem Aufnahmeteil verschoben werden.

Das Griffstück umfasst ein Einstellelement, welches zum Verdrehen quer zur Längsachse des Schiebeteils ausgebildet ist. Das heißt, das Einstellelement kann um die Längsachse, also rotatorisch bewegt werden. Zudem umfasst das Griffstück noch ein Wippelement, welches zwischen dem Einstellelement und dem Schiebeteil angeordnet ist. Das Wippelement kann mit dem Einstellelement zusammen entlang der Längsachse verschoben werden. Allerdings ist das Wippelement im Gegensatz zu dem Einstellelement nicht drehbar. Stattdessen ist das Wippelement rotatorisch ortsfest oder drehfest mit dem Schiebeteil verbunden. Das Wippelement und das Einstellelement bilden zusammen die vorgenannte Klemmeinrichtung aus.

Dabei ist das Einstellelement dazu ausgebildet, durch Verdrehen in eine Blockierrichtung quer zur Längsachse in eine Blockierposition das jeweilige Wippelement in eine Arretierstellung zu dem Schiebeteil zu verschwenken. In der Arretierstellung ist das Verschieben des Griffstücks entlang der Längsachse blockiert. Um die Blockierung zu lösen, ist das Einstellelement weitert ausgebildet, durch Verdrehen in eine von der Blockierrichtung verschiedene Verstellrichtung quer zur Längsachse in eine von der Blockierposition verschiedene Verstellposition das Wippelement in eine Freistellung zu dem Schiebeteil zu verschwenken. In der Freistellung ist das Verschieben des Griffstücks entlang der Längsachse freigegeben.

Somit kann durch das Verdrehen des Einstellelements festgelegt werden, wie tief oder weit das Schiebeteils in den Aufnahmeteil eindringen kann. So kann besonders einfach die Eindringtiefe verstellt oder festgelegt werden. Wird die Vorrichtung zum Beispiel als ein Aspirationsnadelträger eingesetzt, kann durch die Eindringtiefe ein Überstand einer Aspirationsnadel aus einem Tubus, der in dem Schiebeteil geführt ist, vorgegeben sein. Durch die Eindringtiefe kann also festgelegt sein, wie weit die Aspirationsnadel aus dem Tubus herausragt.

Durch die Vorrichtung wird insbesondere eine Einhandbedienung und somit die Handhabbarkeit des Handstücks für ein medizinisches Personal erleichtert. Zudem kann die Gefahr eines ungewollten Verschiebens nach erfolgter Positionierung des Schiebeteils relativ zu dem Aufnahmeteil zumindest weitgehend verhindert werden.

Mit dem aus dem Stand der Technik bekannten Twistlock kann es in bestimmten Situationen passieren, dass die Blockierung ungewollt gelöst wird. Das kann zum Beispiel eintreten, wenn die Vorrichtung für einen medizinischen Eingriff zwischen mehreren verschiedenen Eindringtiefen verstellt werden soll. Dann kann das Griffstück während des Eingriffs verrutschen, was beispielsweise zu einem unerwünschten Nadelüberstand der Aspirationsnadel aus einem Tubus führen kann.

Die US 2018/0317750 A1 offenbart einen Verschluss für einen Griff eines Endoskops. Der Verschluss umfasst eine Schelle mit einem verschwenkbaren Arm, die entlang einer Schiene verschoben und dort befestigt werden kann. Der Arm hat eine Verzahnung und einen Vorsprung, die beim Verschließen in die Verzahnung einer Schiene beziehungsweise in eine Aussparung der Schelle eingreifen.

Die US 2006/0009677 A1 offenbart ein Endoskop, bei dem zwei Instrumententeile über eine Kopplung lösbar miteinander verbunden sind. Die Kopplung umfasst ein magnetisches Element um ein Verschlussteil der Kopplung in einer verschlossenen Position zu halten.

Es ist die Aufgabe der vorliegenden Erfindung, die Handhabung der aus dem Stand der Technik bekannten Vorrichtung zu verbessern.

Lösungen der Aufgabe sind in den unabhängigen Patentansprüchen angegeben. Weitere Ausgestaltungsmöglichkeiten mit zusätzlichen Vorteilen ergeben sich aus den abhängigen Patentansprüchen, der Beschreibung sowie den Figuren.

Die Erfindung basiert auf der Erkenntnis, dass zum Verbessern der Handhabung des bekannten Twistlocks, wie er zuvor beschrieben wurde, ein zusätzlicher Lock- oder Verriegelungsmechanismus ergänzt werden kann. Es geht also darum, die Arretierung oder Blockierung abzusichern. Das kann durch einen zusätzlichen Fixierungs-, Sicherungs- oder Verriegelungsmechanismus des etablierten Twistlocks umgesetzt sein.

Um die zusätzliche Fixierung zu realisieren, umfasst das Griffstück, wie es eingangs beschrieben wurde, zumindest eine Fixiereinrichtung. Das heißt, es kann eine oder es können mehrere Fixiereinrichtungen vorgesehen sein. Die jeweilige Fixiereinrichtung umfasst ein mittels des Einstellelements zu dem Schiebeteil quer zur Längsachse verdrehbares erstes Fixierelement. Des Weiteren umfasst die jeweilige Fixiereinrichtung ein gegen das Verdrehen quer zur Längsachse zu dem Schiebeteil ortsfestes oder drehfestes zweites Fixierelement. In der Blockierposition des Einstellelements, also insbesondere dann, wenn das Verschieben entlang der Längsachse mittels des Wippelements blockiert ist, sind das jeweilige erste und zweite Fixierelement dazu ausgebildet, eine Haltekraft zum Fixieren des Einstellelements gegen das Verdrehen quer zur Längsachse auszuüben.

Anders ausgedrückt, können das erste und zweite Fixierelement durch oder nach dem Verdrehen des Einstellelements so zueinander verstellt werden, dass die gewünschte Haltekraft wirkt oder wirken kann. Zum Beispiel kann das Einstellelement mit der Haltekraft beaufschlagt werden. Dadurch kann das Einstellelement in der Blockierposition gehalten werden. Durch die Haltekraft ist vorzugsweise ein zusätzlicher Kraftaufwand notwendig, um das Einstellelement aus der Blockierposition heraus, zum Beispiel in die Verstellposition oder in eine andere Position zu bewegen. Mittels der Haltekraft kann somit insbesondere das Verdrehen in die Verstellrichtung oder in die Blockierrichtung gesperrt sein.

Vorzugsweise ist die für die Fixierung notwendige oder ausreichende Haltekraft vorgegeben. Das heißt, die Haltekraft kann einen vorgegeben Wert oder Wertebereich aufweisen. Der Wertebereich kann zum Beispiel durch Testversuche ermittelt werden. Insbesondere ist die Haltkraft so gewählt, dass die Einhandbedienung der Vorrichtung weiter möglich bleibt.

Hierdurch ergibt sich der Vorteil, dass die Vorrichtung gegen ein ungewolltes Lösen der Blockierung gesichert ist. Nach erfolgter Positionierung des Griffstücks relativ zu dem Schiebeteil und/oder Aufnahmeteil kann somit ein ungewolltes Verschieben des Griffstücks noch effektiver verhindert werden.

Das genannte Einstellelement kann zumindest zwei verschiedene Positionen oder Stellungen aufweisen. Eine Position kann die vorgenannte Verstellposition sein. In der Verstellposition kann sich das Griffstück in einer Ausgangsstellung befinden. Dabei ist das Griffstück translatorisch verschiebbar. Das Griffstück ist insbesondere nicht blockiert oder arretiert und nicht verriegelt oder fixiert. Die zumindest eine andere Position kann die vorgenannte Blockierposition sein. In der Blockierposition kann sich das Griffstück in einer Nullstellung befinden. In der Blockierposition ist das Griffstück gegen das axiale oder translatorische Verschieben blockiert und fixiert. Das Griffstück ist insbesondere nicht translatorisch verschiebbar.

Das Fixieren erfolgt mittels der Fixierelemente, wie sie zuvor beschrieben wurden. Die Fixierelemente können zum Beispiel eine komplementäre Fixierungsstruktur ausbilden. Das heißt, die Fixierelemente können zum Beispiel eine form- und/oder kraftschlüssige Verbindung ausbilden, um die Blockierposition zu halten. Es kann sich zum Beispiel um eine wie zum Beispiel eine Rastverbindung oder magnetische Verbindung handeln. Somit können die Fixierelemente zusammenwirken, um die Haltekraft bereitzustellen. Das erste Fixierelement kann zum Beispiel von dem Einstellelement umfasst sein. Das zweite Fixierelement kann zum Beispiel von dem Schiebeteil und/oder dem Wippelement umfasst sein.

Zum Lösen der Fixierung und/oder der Blockierung muss diese Haltekraft überwunden werden. Das heißt, es ist eine zusätzliche Bedienkraft bei der Bedienung des Einstellelements aufzuwenden, um die Fixierung und insbesondere auch die Blockierung aufzuheben, so dass das Griffstück zum Beispiel wieder translatorisch verschiebbar ist.

Die Vorrichtung, insbesondere das Griffstück, kann weitere Aspekte oder Merkmale umfassen, wie sie aus dem eingangs beschriebenen Stand der Technik, insbesondere der EP 2 531 122 B1, bereits bekannt sind. Zum Beispiel können das Wippelement oder das Schiebeteil jeweils zugeordnete Anlageflächen umfassen, die einen Reibungsbelag oder Bremsbelag aufweisen. Somit kann die Blockierung oder Ausnutzung von Reibung zwischen dem Wippelement und dem Schiebeteil erreicht werden. Alternativ können das Wippelement und das Schiebeteil eine jeweilige Zahneinrichtung ausbilden, mittels welcher in der Arretierstellung jeweilige Zahnelemente in zugeordnete Zahnelementaufnahmen eingreifen.

Das jeweilige Wippelement kann zum Beispiel mittels eines Lagerzapfens in einer entlang der Längsachse verlaufenden Lagernut des Schiebeteils angeordnet sein. Dadurch kann das jeweilige Wippelement verdrehsicher (ortsfest) geführt sein.

Das Einstellelement kann zum Beispiel einen oval-symmetrisch oder sichelförmig geformten Öffnungsbereich umfassen. Der Öffnungsbereich kann ein das Wippelement aufnehmender Innenbereich des Einstellelements sein. Dadurch kann das Verschwenken des Wippelements bewirkt werden.

Des Weiteren kann das Schiebeteil zum Beispiel als Skalenkolben ausgebildet sein. Das heißt, das jeweilige Schiebeteil kann eine oder mehrere Markierungen (Skala) oder Skaleneinteilungen enthalten.

Im Folgenden sind Ausführungsformen der Erfindung beschrieben durch die sich weitere Vorteile ergeben.

In den folgenden Ausführungsformen geht es zunächst darum, wie die Fixierung, also der Lock-Mechanismus, realisiert sein kann. Beispielsweise kann die Fixiereinrichtung mittels eines aktiven oder eines passiven oder einer Kombination aus einem aktiven und passiven Fixiermechanismus konzipiert sein.

Die aktive Fixierung kann gemäß einer Ausführungsform der Erfindung zum Beispiel dadurch umgesetzt sein, dass das erste Fixierelement einen Sperrkörper ausbildet, und das zweite Fixierelement eine Ausnehmung oder Aussparung zum Aufnehmen des Sperrkörpers ausbildet. Der Sperrkörper ist dazu ausgebildet, in der Blockierposition des Einstellelements mittels einer Bedienhandlung einer Bedienperson in eine Fixierstellung bewegt oder verstellt zu werden. In der Fixierstellung ist die Haltekraft maximal.

Zum Lösen der Fixierung, also zum Entriegeln kann analog dazu vorgesehen sein, dass Sperrkörper dazu ausgebildet ist, in der Fixierstellung mittels einer Bedienhandlung einer Bedienperson in eine Entriegelungsstellung bewegt oder verstellt zu werden. In der Entriegelungsstellung ist die Haltekraft minimal, insbesondere reduziert, vorzugsweise gleich null (nullifiziert).

Das heißt, es ist eine zu dem Verdrehen zusätzliche manuelle Bedienung notwendig, um das Einstellelement zu fixieren oder zu entriegeln. Mittels der Bedienhandlung kann das erste Fixierelement zwischen zumindest zwei Stellungen verstellt oder bewegt werden. Eine Stellung ist die Fixierstellung. Eine weitere der Stellungen ist die Entriegelungsstellung.

In der Fixierstellung kann der Sperrkörper formschlüssig zum Beispiel mittels Rastverbindung oder Steckverbindung mit der Ausnehmung verbunden sein. Das heißt, der Sperrkörper ist in der Ausnehmung aufgenommen oder in diese eingesteckt.

Der Sperrkörper kann zum Beispiel als Knopf oder Stift ausgebildet sein. Zusätzlich kann der Sperrkörper zum Beispiel ein oder mehrere Federelemente umfassen, um ein leichteres Lösen der Fixierung zu ermöglichen.

In einer konkreten Ausgestaltung kann in den Twistlock-Drehmechnismus (Einstellelement) ein per manueller Betätigung auszulösender Stift oder Knopf (Sperrkörper) eingearbeitet sein. Dieser kann zum Beispiel innenliegend mit einer Spiralfeder ausgestattet sein. Zur Arretierung wird der Twistlock-Drehmechnismus, wie zuvor beschrieben, verdreht. Zur Sicherung der Arretierung wird der Stift oder Knopf per zusätzlicher, manueller Bedienhandlung nach unten gedrückt. Eine innenliegende Kontur des Stiftes oder des Knopfs greift in die dafür vorgesehene Aussparung (Ausnehmung) ein, die zum Beispiel in das Schiebeteil eingebracht sein kann. Zum Lösen der Fixierung und der Arretierung muss somit vor der Drehbewegung des Twistlocks der Stift oder Knopf erneut gedrückt oder gezogen (push-push oder push-pull Bedienung) werden, damit dieser aus der vorgesehenen Aussparung entfernt wird. Erst dann wird die Drehmöglichkeit, also das Verdrehen des Twistlocks wieder freigegeben. Insgesamt kann so die aktive Fixierung umgesetzt sein.

Umsetzungsbeispiele für die vorgenannte passive Fixierung sind in den folgenden Ausführungsformen angegeben.

Gemäß einer Ausführungsform kann die passive Fixierung dadurch realisiert sein, dass das Einstellelement dazu ausgebildet ist, durch das Verdrehen quer zur Längsachse in die Blockierposition, das erste Fixierelement in eine Fixierstellung zu dem zweiten Fixierelement zu bewegen, in welcher die Haltekraft maximal ist. Die Fixierung erfolgt somit automatisch durch das Verdrehen des Einstellelements. Dadurch ist die Fixierung ein zusätzlicher Effekt, der bei der Blockierung herbeigeführt werden kann.

Zum Lösen der Fixierung, also zum Entriegeln kann analog dazu eine entsprechende passive Entriegelung vorgesehen sein. Das heißt, das Einstellelement kann dazu ausgebildet ist, durch das Verdrehen quer zur Längsachse beispielsweise in die Verstellposition oder eine zu der Blockierposition verschiedene Position, das erste Fixierelement in eine Entriegelungsstellung zu dem zweiten Fixierelement zu bewegen, in welcher die Haltekraft minimal oder nullifiziert ist.

Mittels des Verdrehens des Einstellelements können die Fixierelemente somit zwischen den zuvor beschriebenen zumindest zwei Stellungen verstellt werden, nämlich der Fixierstellung und der Entriegelungsstellung.

Gemäß einer weiteren Ausführungsform können das erste und das zweite Fixierelement ein magnetisches Material umfassen und zueinander ein gegenpoliges Magnetfeld ausbilden. Die Fixierung kann somit durch Kraftschluss, insbesondere eine magnetische Verbindung, der Fixierelemente erfolgen. In der Fixierstellung kann eine Anziehungskraft oder Magnetkraft der Fixierelemente so groß sein, dass sich die Fixierelemente gegenseitig anziehen. In der Entriegelungsstellung erfolgt hingegen keine Anziehung. Die Fixierelemente können Magnete, insbesondere Permanentmagnete ausbilden.

Konkret könnte die magnetische Fixierung zum Beispiel durch von dem Einstellelement umfasste Magnete und einen von dem Skalenkolben oder dem Wippelement umfassten gegenpoligen Magneten realisiert sein. Insbesondere kann in das Schiebeteil zum Beispiel eine Magnetschiene eingearbeitet sein, die entlang der Längsachse über die gesamte Länge des Schiebeteils verläuft. Die Magnetschiene kann zum Beispiel im Volumen oder an der Oberfläche des Skalenkolbens montiert oder fixiert sein. Ebenso kann von dem Twistlock-Drehmechnismus ein gegenpoliger Magnet eingearbeitet, also an der Oberfläche oder in das Volumen eingelassen sein.

Baulich, also bezüglich der Position ist der dem Twistlock zugeordnete Magnet im geöffneten Zustand (Verstellposition) mit großem Abstand, insbesondere rotatorisch gesehen beabstandet zu der Magnetschiene im Skalenkolben positioniert. Durch die Drehbewegung des Twistlocks in die Blockierposition wird die Position des Magneten so manipuliert, dass dieser deckungsgleich über der Magnetschiene des Skalenkolbens steht. So kann sich die maximale Magnetkraft ausbilden. Dadurch können eine mechanische Blockierung des Twistlocks unterstützt und ein ungewolltes Lösen des Twistlocks verhindert werden.

Gemäß einer weiteren Ausführungsform ist die passive Verriegelung dadurch realisiert, dass das erste Fixierelement als Wälzlager mit zumindest einem Wälzkörper ausgebildet ist. Das zweite Fixierelement ist als eine Ausnehmung zum Aufnehmen des Wälzkörpers in der Fixierstellung ausgebildet. In der Fixierstellung kann der Wälzkörper somit in der Ausnehmung angeordnet oder angebracht sein. Es kann eine formschlüssige Verbindung realisiert sein.

Das Wälzlager kann zum Beispiel ein an sich bekanntes Kugellager sein. Entsprechend kann der Wälzkörper zum Beispiel eine Kugel ausbilden. Der Wälzkörper kann sich beim Verdrehen des Einstellelements entlang der Oberfläche des Schiebeteils bewegen, vorzugsweise im Wesentlichen reibungsfrei. Um den Wälzkörper in die Ausnehmung zu bewegen, kann der Wälzkörper zum Beispiel federbelastet sein. Die Ausnehmung oder Aussparung kann eine zu dem Wälzkörper komplementär ausgebildete Vertiefung in dem Schiebeteil sein, in die der Wälzkörper einrasten oder eingleiten kann.

In einer konkreten Umsetzung kann der Twistlock zum Beispiel eines oder mehrere Kugellager, insbesondere aus Metall, umfassen, die in geometrische Taschen (Ausnehmung) des Skalenkolbens tauchen und dadurch zusätzliche Kräfte zum Lösen des Twistlock-Drehmechnismus notwendig machen. Dazu können zum Beispiel einzelne oder mehrere Kugellager in die dem Skalenkolben zugewandte Innenseite des Einstellelements eingearbeitet oder eingelassen werden. Im gelösten Zustand des Twistlock-Drehmechnismus (Verstellposition) kann das jeweilige Kugellager auf der geschlossenen Oberfläche des Skalenkolbens aufliegen. Beim Verdrehen des Twistlocks können die Kugeln des Kugellagers vorzugsweise ohne Reibung auf der Oberfläche rollen oder rotieren. In der Blockierposition, also beim Arretieren des Twistlocks mittels der Drehung, können die Kugeln der Kugellager in entsprechend in die Oberfläche des Skalenkolbens eingelassene Taschen oder Aussparungen fallen oder eingreifen. Dadurch wird die zusätzliche Verriegelung realisiert. Zum Lösen der Arretierung ist kein zusätzlicher Lösemechanismus, also keine zusätzliche Bedienhandlung notwendig. Es kann zum Beispiel genügen, das Einstellelement mit vergrößertem Kraftaufwand zu verdrehen, um den Formschluss zu überwinden. Dazu kann die jeweilige Ausnehmung zum Beispiel an dabei Geometrie des jeweiligen Wälzkörpers, also zum Beispiel der Kugel, angepasst sein.

Wie zuvor erwähnt, kann das Griffstück zum Beispiel eine oder mehrere der Fixiereinrichtungen der zuvor beschriebenen Arten umfassen. Zum Beispiel kann das Griffstück mehrere, also zwei oder mehr gleichartige Fixiereinrichtungen aufweisen. Mit gleichartig ist gemeint, dass die Fixiereinrichtungen die gleichen Fixierelemente aufweisen und somit einen gleichen Fixiermechanismus, wie er zuvor beschrieben wurden, realisieren. Alternativ kann das Griffstück zum Beispiel mehrere, also zwei oder mehr verschiedenartige Fixiereinrichtungen umfassen. Die Fixiereinrichtungen können zueinander somit verschiedene Fixierelemente aufweisen und unterschiedliche Fixiermechanismen, wie sie zuvor beschrieben wurden, realisieren.

Zum Beispiel kann eine Kombination aus einer passiven und aktiven Verriegelung umgesetzt sein. Es kann zum Beispiel vorgesehen sein, die Fixierung passiv erfolgt, während das Entriegeln aktiv durchgeführt wird. So können die Fixierelemente durch Verdrehen des Einstellelements quer zur Längsachse in die Blockierposition, wie für die passive Verriegelung beschrieben, automatisch in die Fixierstellung bewegt werden. Um die Fixierung zu lösen, ist in der Blockierposition des Einstellelements hingegen die zusätzliche Bedienhandlung, wie sie für die aktive Verriegelung beschrieben wurde, notwendig, um die Fixierelemente von der Fixierstellung in die Entriegelungsstellung zu bewegen. Konkret könnte zum Beispiel vorgesehen sein, dass beim Verriegeln ein Verrieglungskörper, also zum Beispiel ein vorgenannter Wälzkörper in eine vorgesehene Tasche (Ausnehmung) des Skalenkolbens fällt, der zum Lösen jedoch aktiv gelöst werden muss. Das heißt, der Verrieglungskörper muss zum Beispiel bewusst hochgezogen, verschoben oder gedrückt werden.

Gemäß einer weiteren Ausführungsform bilden das Einstellelement und das Wippelement eine Führungsstruktur zueinander, insbesondere relativ zueinander aus. Die Führungsstruktur gibt einen Führungsverlauf zumindest für das Verdrehen des Einstellelements quer zur Längsachse relativ zu dem Wippelement vor. Das Einstellelement ist entlang des Führungsverlaufs in die jeweilige Position, also zum Beispiel die vorgenannte Verstell- und Blockierposition oder weitere im späteren näher beschriebene Positionen verstellbar.

Der Führungsverlauf oder die Führungsstruktur gibt somit eine Bewegungsrichtung oder Bahnkurve für das Einstellelement vor. Dieser diskrete Führungsverlauf kann eine besonders einfache Bedienung durch die Bedienperson ermöglichen.

Die Führungsstruktur kann zum Beispiel durch zwei Führungskörper gebildet sein. Die Führungskörper können miteinander gekoppelt, insbesondere formschlüssig miteinander verbunden sein. Der erste Führungskörper kann zum Beispiel ein Knopf oder ein Stift oder ein Zapfen sein. Der zweite Führungskörper kann zum Beispiel eine Führungsnut oder Führungsschiene. Der erste Führungskörper kann in den zweiten Führungskörper eingreifen. Der zweite Führungskörper kann den Führungsverlauf vorgeben. Die Führungskörper können relativ zueinander bewegbar sein. Der erste Führungskörper kann zum Beispiel drehfest zu dem Schiebeteil ausgebildet sein. Zum Beispiel kann der erste Führungskörper von dem Schiebeteil oder dem jeweiligen Wippelement umfasst sein. Der zweite Führungskörper kann verdrehbar zu dem Schiebeteil ausgebildet sein. Zum Beispiel kann der zweite Führungskörper von dem Einstellelement umfasst sein.

In diesem Zusammenhang ist in einer weiteren Ausführungsform vorgesehen, dass die Führungsstruktur einen der Blockierposition zugeordneten Führungsverlaufsabschnitt umfasst, welcher entlang der Längsachse verläuft. Zum Lösen der Fixierung beziehungsweise der Blockierung ist das Einstellelement entlang des Führungsverlaufsabschnitts relativ zu dem jeweiligen Wippelement verschiebbar.

Das heißt, durch den Führungsverlauf kann eine Möglichkeit zum besonders sicheren Fixieren des Griffstücks vorgesehen sein. Dabei ist zum Entriegeln eine zusätzliche Bedienbewegung, insbesondere ein Verschieben des Einstellelements notwendig, um die Fixierung zu lösen. Insbesondere ist die Entriegelung durch das Verschieben des Einstellelements aus der Blockierposition heraus entlang der Längsachse einleitbar. Die Verschiebebewegung muss bewusst durchgeführt werden, wodurch eine Hinweis- oder Warnfunktion für die Bedienperson realisiert ist.

Dazu ist der Führungsverlauf in der Blockierposition quer zur Blockierrichtung entlang der Längsachse versetzt ausgeführt. Es ergibt sich somit ein stufenförmiges oder treppenförmiges Verlaufsprofil der Führungsstruktur, insbesondere des zweiten Führungskörpers.

Alternativ dazu kann ein besonders einfacher Entriegelungsmechanismus dadurch realisiert sein, dass zum Entriegeln das Einstellelement zum Beispiel zum Verdrehen aus der Blockierposition heraus in die Verstellrichtung ausgebildet sein. Das heißt, das Einstellelement kann durch Verdrehen in die Verstellrichtung direkt aus der Blockierposition in die Verstellposition gebracht oder bewegt werden. Dazu kann die Führungsstruktur zum Beispiel einen geradlinigen Führungsverlauf vorgeben.

Zusätzlich oder alternativ der Führungsstruktur kann ein weiterer Entriegelungsmechanismus vorgesehen sein, der eine besonders sichere Fixierung und eine besonders bewusste Entriegelung umsetzt.

Dazu ist gemäß einer weiteren Ausführungsform das Einstellelement zum Lösen der Fixierung dazu ausgebildet, durch Verdrehen aus der Blockierposition in die Blockierrichtung eine von der Blockierposition und der Verstellposition verschiedene Entriegelungsposition einzunehmen. Dadurch ist das Einstellelement ausgebildet, das erste und zweite Fixierelement in die vorgenannte Entriegelungsstellung zu bewegen, in welcher die Haltekraft im Vergleich zu der Fixierstellung reduziert, vorzugsweise minimal, ist. Das jeweilige Wippelement behält in der Entriegelungsposition des Einstellelements die Arretierstellung jedoch bei.

Das heißt, in der Entriegelungsposition ist das Griffstück weiterhin gegen das Verschieben entlang der Längsachse blockiert. Somit ist das Griffstück nicht verstellbar. Das Griffstück ist aber nicht mehr verriegelt oder fixiert. Das heißt, das Verdrehen des Einstellelements ist wieder ohne zusätzlichen Kraftaufwand möglich.

Für die Entriegelung ist das Einstellelement somit in dieselbe Richtung weiterzudrehen wie für die Blockierung, nämlich in die Blockierrichtung und nicht in die Verstellrichtung. Dadurch ist eine zusätzliche bewusste Bedienhandlung notwendig, um die Fixierung und anschließend auch die Blockierung zu lösen. Somit ergibt sich eine besonders sichere Blockierung und Fixierung des Griffstücks gegen das Verschieben.

In diesem Zusammenhang ist gemäß einer weiteren Ausführungsform vorgesehen, dass das Griffstück eine Entriegelungseinrichtung umfasst. Die Entriegelungseinrichtung ist zwischen dem Schiebteil und dem Einstellelement angeordnet. Die Entriegelungseinrichtung ist dazu ausgebildet, durch oder beim Verdrehen des Einstellelements quer zur Längsachse die jeweiligen Fixierelemente in der Entriegelungsstellung zu halten.

Vorzugsweise kann die Entriegelungseinrichtung die jeweiligen Fixierelemente immer nur dann in der Entriegelungsstellung halten, wenn sich das Einstellelement außerhalb der Blockierposition befindet. Somit kann außerhalb der Blockierposition die Fixierung verhindert werden. Dazu können mittels der Entriegelungseinrichtung die Fixierelemente zum Beispiel axial (entlang der Längsachse) in einem vorgegebenen Abstand gehalten oder zueinander angeordnet werden.

In den folgenden Ausführungsformen wird näher darauf eingegangen, wie die Entriegelungseinrichtung konkret realisiert sein kann.

Gemäß einer Ausführungsform umfasst die Entriegelungseinrichtung ein erstes Entriegelungselement. Die erste Entriegelungseinrichtung ist gegen das Verdrehen quer zur Längsachse zu dem Schiebeteil ortsfest, also drehfest angeordnet. Somit kann das Einstellelement kann relativ zu dem ersten Entriegelungselement rotatorisch verdrehbar sein.

Das Einstellelement und das erste Entriegelungselement bilden eine Abstandshaltestruktur aus. Die Abstandshaltestruktur ist dazu ausgebildet, beim Verdrehen des Einstellelements quer zur Längsachse, also insbesondere außerhalb der Blockierposition, das Einstellelement und das erste Entriegelungselement entlang der Längsachse relativ zueinander in einem vorgegebenen Abstand zueinander zu halten. Dadurch ist die Entriegelungsstellung der Fixierelemente realisiert. Das heißt, der Abstand kann auf die Fixierelemente übertragen sein.

Zudem umfasst die Abstandhaltestruktur einen Verbindungsmechanismus, mittels welchem das erste Entriegelungselement und das Einstellelement in der Blockierposition formschlüssig verbunden sind und dabei der vorgegebene Abstand reduziert ist. Durch die formschlüssige Verbindung ist die Fixierstellung der Fixierelemente realisiert.

Somit können das Einstellelement und das erste Entriegelungselement translatorisch zueinander verschiebbar sein. Mit dem Abstand ist vorliegend gemeint, dass das Einstellelement und das erste Entriegelungselement außerhalb der Blockierposition entlang der Längsachse relativ zueinander verschoben, insbesondere auseinandergezogen, gehalten sind. Anders ausgedrückt, können die Fixierung und das Entriegeln dadurch realisiert werden, dass das Einstellelement und das erste Entriegelungselement relativ zueinander entlang der Längsachse verschoben, insbesondere auseinandergezogen werden. Das erste Entriegelungselement kann dabei im Überlappungsbereich mit dem Einstellelement weiter in Richtung Rand des Überlappungsbereichs oder zumindest bereichsweise aus dem Überlappungsbereich bewegt werden. Es brauchst somit keine sichtbare Lücke zwischen Einstellelement und erstem Entriegelungselement entstehen. Durch das Verschieben können die Fixierelemente auseinandergezogen (Entriegelungsstellung) oder in Verbindung gebracht (Fixierstellung) werden.

Die Abstandshaltestruktur kann durch zwei komplementäre Abstandshaltekörper gebildet sein, von denen einer von dem Einstellelement und der andere von dem ersten Entriegelungselement umfasst ist. Zum Beispiel können die Abstandshaltekörper ein Abstützteil und ein Halteteil ausbilden. Außerhalb der Blockierposition können dieses gegeneinander abgestützt sein. In der Blockierposition kann das Abstützteil in das Halteteil eingreifen, so dass sich der Formschluss bildet. Das Abstützteil kann zum Beispiel als Zapfen oder Vorsprung oder Nase ausgebildet sein. Das Halteteil kann eine Auflagefläche für das Abstützteil bilden, die quer zur Längsachse verläuft. Beim Verdrehen kann das Halteteil entlang der Auflagefläche gleiten. Die Auflagefläche kann eine mit der Blockierposition korrespondierende Nut oder Aufnahme umfassen, in die das Halteteil eingepasst sein kann. Dadurch kann der Verbindungsmechanismus realisiert sein. Der Verbindungsmechanismus kann eine zusätzliche Fixierung zu den Fixierelemente ermöglichen.

Die geometrische Form von Zapfen und Nut kann so gewählt sein, dass das Verdrehen blockiert ist. Es kann somit eine zusätzliche Bedienhandlung, wie zum Beispiel ein auseinanderziehen notwendig sein, um die formschlüssige Verbindung zu lösen. Ein entsprechender Bewegungsverlauf kann zum Beispiel durch den parallel zur Längsachse verlaufenden Führungsverlaufsabschnitt der Führungsstruktur vorgegeben sein.

Gemäß einer weiteren Ausführungsform umfasst die Entriegelungseinrichtung ein Signalisierungselement, welches durch Verschieben des Einstellelements und des ersten Entriegelungselements nur in der Blockierposition zumindest bereichsweise entlang der Längsachse über das Einstellelement zum Signalisieren des Blockierens und Fixierens hinausragt.

Somit ist das Signalisierungselement insbesondere nur in der Blockierposition sichtbar. Außerhalb der Blockierposition ist das Signalisierungselement vorzugsweise von dem Einstellelement und/oder dem ersten Entriegelungselement vollständig überdeckt oder überlappt.

Das Signalisierungselement kann dazu im blockierten Zustand des Griffstücks rotational und translatorisch ortsfest zu dem Schiebeteil angeordnet sein. Das Signalisierungselement kann zum Beispiel eine Signalfarbe, wie zum Beispiel rot, umfassen. So kann die Blockierung und/oder Fixierung besonders eindeutig angezeigt werden. Das Signalisierungselement kann beispielsweise einen Anschlag, also einen Anlagebereich, für das Aufnahmeteil ausbilden.

Gemäß einer weiteren Ausführungsform weist die Entriegelungseinrichtung ein zweites Entriegelungselement auf. Das zweite Entriegelungselement ist gegen das Verschieben entlang der Längsachse relativ zu dem Einstellelement ortsfest angeordnet. Das heißt, das zweite Entriegelungselement ist insbesondere nur zusammen mit dem Einstellelement verschiebbar. Das erste und zweite Entriegelungselement bilden eine Verschiebestruktur aus. Die Verschiebestruktur ist dazu ausgebildet, beim Verdrehen des Einstellelements quer zur Längsachse aus der Blockierposition heraus in die Entriegelungsposition, das Einstellelement und das erste Entriegelungselement entlang der Längsachse relativ zueinander in den vorgegebenen Abstand zueinander zu verschieben.

Das heißt, das zweite Entriegelungselement kann genutzt werden, um den Formschluss des Verbindungsmechanismus der Abstandshaltestruktur des Einstellelements und des ersten Entriegelungselements zu lösen.

Die Verschiebestruktur kann durch zwei komplementäre Verschiebekörper gebildet sein, von denen einer von dem ersten und der andere von dem zweiten Entriegelungselement umfasst ist. Zum Beispiel können die Verschiebekörper analog zu den vorgenannten Abstandshaltekörpern ein Abstützteil und ein Halteteil ausbilden. Außerhalb der Blockierposition können dieses gegeneinander abgestützt sein. In der Blockierposition kann das Abstützteil in das Halteteil eingreifen, so dass sich ein Formschluss bildet. Das Abstützteil kann zum Beispiel als Zapfen oder Vorsprung oder Nase ausgebildet sein. Das Halteteil kann eine Auflagefläche für das Abstützteil bilden, die quer zur Längsachse verläuft. Beim Verdrehen kann das Halteteil entlang der Auflagefläche gleiten. Die Auflagefläche kann eine mit der Blockierposition korrespondierende Nut oder Aufnahme umfassen, in die das Halteteil eingepasst sein kann.

Vorzugsweise ist die geometrische Form von Zapfen und Nut so gewählt, dass das Verschieben automatisch durch das Verdrehen des Einstellelements, vorzugsweise nur beim Verdrehen in die Blockierrichtung, erfolgt. Dazu können die Verschiebekörper eine jeweilige Anlagefläche umfassen, mittels welcher die Verschiebekörper in der Blockierposition aneinander anliegen, die quer zur Längsachse schräg gestellt ist. Es ergibt sich eine schiefe Ebene oder Rampe. Dadurch können die Verschiebekörper beim Verdrehen des Einstellelements besonders einfach aneinander entlanggleiten. Dadurch werden das Einstellelement und erstes Entriegelungselement auseinanderbewegt bis der gewünschte Abstand erreicht ist.

Gemäß einer weiteren Ausführungsform umfassen das Einstellelement und das zweite Entriegelungselement eine Koppelstruktur zum zumindest abschnittsweisen Mitführen des zweiten Entriegelungselements beim Verdrehen des Einstellelements quer zur Längsachse. Dabei ist die Koppelstruktur ausgebildet, die Abstandshaltestruktur und die Verschiebestruktur beim Verschieben des Einstellelements aus der Entriegelungsposition in die Verstellposition quer zur Längsachse zu versetzen und dadurch den vorgegebenen Abstand beim Verdrehen in die Verstellrichtung zu halten.

Durch das rotatorische Versetzen kann verhindert werden, dass der Verbindungsmechanismus des Einstellelements und der jeweiligen Entriegelungseinrichtung greifen, wenn das Einstellelement zurück in die Ausgangsstellung, also in die Verstellposition, gedreht wird.

Durch die Koppelstruktur ist das zweite Entriegelungselement somit ausgebildet, im gekoppelten Zustand durch Verdrehen des Einstellelements quer zur Längsachse relativ zu dem Schiebeteil verdreht zu werden. Das Einstellelement führt das zweite Entriegelungselement mit. Im ungekoppelten Zustand wird das zweite Entriegelungselement hingegen nicht mitgeführt. Es bleibt drehfest zu dem Schiebeteil.

Die Koppelstruktur kann zum Beispiel einen Mitnahmekörper und einen Mitführkörper umfassen, von denen einer am zweiten Entriegelungselement und einer am Einstellelement angeordnet ist. In einer Koppelstellung, also im gekoppelten Zustand, liegen die Körper aneinander an oder greifen ineinander ein. Dabei kann der Mitführkörper den Mitnahmekörper mit einer Kraft oder mit einem Impuls beaufschlagen, wodurch die rotatorische Bewegung um die Längsachse realisiert ist.

Somit kann die Entriegelungseinrichtung neben der Entriegelungsfunktion (ermöglichen der Entriegelungsstellung) auch eine Verriegelungsfunktion (ermöglichen und/oder halten der Fixierstellung) realisieren. Die Entriegelungseinrichtung kann deshalb auch als Verriegelungseinrichtung bezeichnet werden. Analog können die Entriegelungselemente auch als Verriegelungselemente bezeichnet werden.

Ein weiterer Aspekt der Erfindung betrifft ein medizinisches Handstück für ein medizinisches Gerät. Das Handstück kann zum Beispiel von dem medizinischen Gerät umfasst oder diesem zugeordnet sein. Das medizinische Handstück umfasst eine erste und eine zweite Vorrichtung der vorgenannten Art. Die erste Vorrichtung ist dabei ausgebildet, die Eindringtiefe eines ersten rohrförmigen Schiebeteiles in einem ersten Aufnahmeteil festzulegen. Die zweite Vorrichtung ist dazu ausgebildet, die Eindringtiefe eines zweiten rohrförmigen Schiebeteiles in einem zweiten Aufnahmeteil festzulegen. Die erste und zweite Vorrichtung sind derart hintereinander angeordnet, dass der erste Schiebeteil der ersten Vorrichtung zumindest zum Teil als zweiter Aufnahmeteil für das zweite Schiebeteil der zweiten Vorrichtung ausgebildet ist. Das erste Aufnahmeteil und das zweite Schiebeteil weisen zudem eine jeweilige Verbindungsschnittstelle zum Verbinden von Elementen des medizinischen Geräts auf.

Dadurch kann ein besonders einfach und sicher zu handhabendes Handstück für ein medizinisches Gerät bereitgestellt werden. Das Handstück kann durch die beiden Vorrichtungen besonders sicher in der jeweiligen Einstellposition, also mit der gewünschten Eindringtiefe, gehalten werden. Somit kommt es im Zuge von Einstellvorgängen zu keinen Irritationen für das Handhabungspersonal.

Das zweite Aufnahmeteil des Handstücks kann vorzugsweise mit dem Griffstück der zweiten Vorrichtung verbunden sein. Entsprechend können das zweite Aufnahmeteil und das Griffstück gemeinsam translatorisch entlang der Längsachse bewegbar sein. Hierdurch ergibt sich der Vorteil, dass die Länge des Handstücks an verschiedene Arten von medizinischen Geräten, insbesondere an Endoskope, angepasst werden kann. Das Handstück ist somit universell einsetzbar.

Im Gegensatz dazu kann das erste Aufnahmeteil beispielsweise separat zu dem Griffstück der ersten Vorrichtung ausgebildet sein. Somit kann das Griffstück relativ zu dem ersten Aufnahmeteil und ersten Schiebeteil translatorisch, also entlang der Längsachse, in Längsrichtung bewegt werden. Das Griffstück kann somit als Stopper für den ersten Aufnahmeteil genutzt werden.

Das Handstück kann weitere Aspekte und Merkmale und umfassen, wie sie bereits im Stand der Technik, insbesondere der EP 2 531 122 B1 beschrieben worden sind.

Zum Beispiel kann das erste Aufnahmeteil ein Anschlusselement zum Verbinden oder Anschließen einer medizinischen Hohl- oder Aspirationsnadel umfassen. Die Hohl- oder Aspirationsnadel kann dabei im verbundenen Zustand von dem medizinischen Handstück umhüllt sein. Das heißt, die Nadel kann sich durch das erste Aufnahmeteil, das erste Schiebeteil und den daran anschließenden zweiten Aufnahmeteil und das zweite Schiebeteil hindurch erstrecken. Insbesondere kann dann das erste Aufnahmeteil dazu ausgebildet sein, durch das Verschieben entlang der Längsachse den Überstand der Aspirationsnadel aus einem Tubus vorzugeben. Dazu kann das erste Aufnahmeteil zum Beispiel für das translatorische Verschieben separat zu dem Griffstück der zweiten Vorrichtung ausgebildet sein.

Das zweite Schiebeteil kann zum Beispiel an seinem distalen Ende ebenfalls ein Verbindungselement zum Verbinden oder Anschließen oder Koppeln mit einem Verbindungselement eines weiteren medizinischen Geräts, insbesondere eines Arbeitskanals eines Endoskops, aufweisen. Am proximalen Endbereich des ersten Aufnahmeteils kann ebenfalls ein Verbindungselement angeordnet sein. Über dieses Verbindungselement kann zum Beispiel ein weiteres medizinisches Gerät, wie zum Beispiel eine Spritze oder eine Absaugeinrichtung oder eine Spüleinrichtung, angeschlossen werden.

Das Aufnahmeteil kann vorzugsweise als Griffteil Ausgangsstellung sein. Dies erleichtert die Handhabbarkeit des Griffstücks bei der Einstellung der beiden Schiebeteile.

Die Erfindung umfasst auch die Kombinationen der beschriebenen Ausführungsformen. Zudem sind von der Erfindung auch Weiterbildungen des erfindungsgemäßen Handstücks umfasst, die Merkmale aufweisen, wie sie bereits im Zusammenhang mit den Weiterbildungen der erfindungsgemäßen Vorrichtung beschrieben worden sind. Aus diesem Grund sind die entsprechenden Weiterbildungen des erfindungsgemäßen Handstücks hier nicht noch einmal beschrieben.

Im Folgenden sind Ausführungsbeispiele der Erfindung beschrieben. Hierzu zeigt:
- Fig. 1: Eine schematische Darstellung einer Vorrichtung zum Festlegen einer Eindringtiefe eines rohr- oder stangenförmigen Schiebeteils in einen Aufnahmeteil gemäß einer beispielhaften Ausführungsform;
- Fig. 2: eine schematische Darstellung der Vorrichtung mit einem Griffstück, dass sich in einem Ausgangszustand befindet;
- Fig. 3: eine schematische Darstellung der Vorrichtung, in der sich das Griffstück in einem Nullzustand befindet;
- Fig. 4: eine schematische Darstellung der Vorrichtung mit dem Griffstück in einer ersten Stellung beim Bringen des Griffstücks von dem Nullzustand in einen Entriegelungszustand;
- Fig. 5: eine schematische Darstellung der Vorrichtung mit dem Griffstück in einer zweiten Stellung beim Bringen des Griffstücks von dem Nullzustand in einen Entriegelungszustand;
- Fig. 6: eine schematische Darstellung der Vorrichtung, in der das Griffstück sich in dem Entriegelungszustand befindet;
- Fig. 7: eine schematische Darstellung der Vorrichtung mit dem Griffstück in einer ersten Stellung beim Bringen des Griffstücks von dem Entriegelungszustand in den Ausgangszustand; und
- Fig. 8: eine schematische Darstellung der Vorrichtung, in der sich das Griffstück erneut in dem Ausgangszustand befindet.

Die im Folgenden näher beschriebenen Ausführungsbeispiele sind als bevorzugte Ausführungsformen der Erfindung anzusehen. Die in den Ausführungsbeispielen beschriebenen Komponenten der Ausführungsformen stellen jeweils einzelne, unabhängig voneinander zu betrachtende Merkmale der Erfindung dar. Diese Merkmale bilden die Erfindung jeweils auch unabhängig voneinander weiter. Sie sind deshalb auch einzeln oder in einer anderen als der gezeigten Kombination als Bestandteil der Erfindung anzusehen. Zudem können weitere der bereits zuvor beschriebenen Merkmale der Erfindung die beschriebenen Ausführungsformen ergänzen.

Für funktionsgleiche Elemente wurden in den Figuren jeweils dieselben Bezugszeichen vergeben.

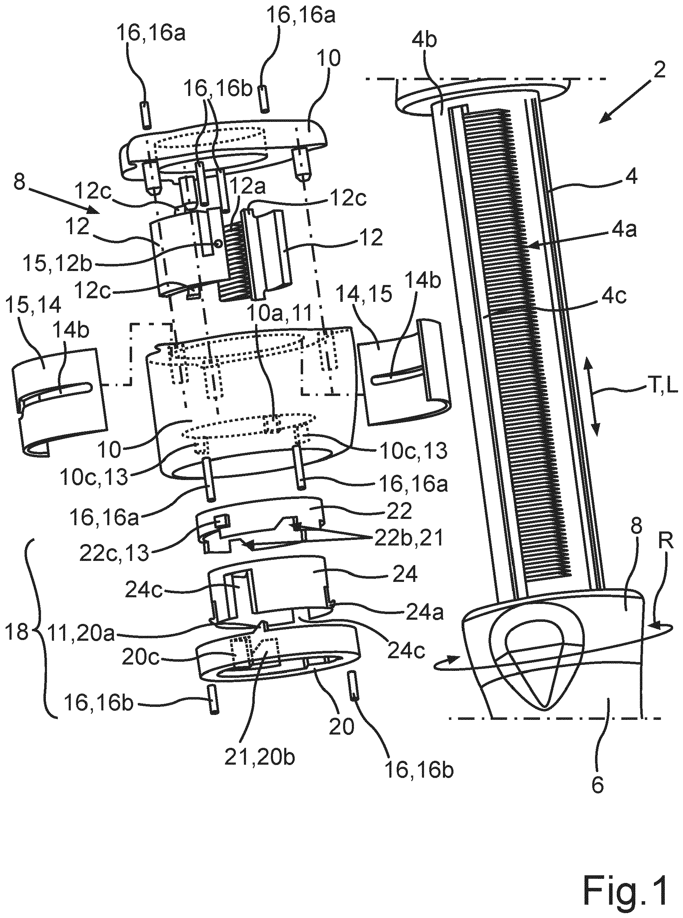

Fig. 1 zeigt eine schematische Darstellung einer Vorrichtung 2. Die Vorrichtung wird zum Festlegen einer Eindringtiefe eines rohr- oder stangenförmigen Schiebeteils 4 in einen Aufnahmeteil 6 eingesetzt. Dazu umfasst die Vorrichtung 2 neben dem Schiebeteil 4 und dem Aufnahmeteil 6 ein Griffstück 8, welches den Schiebeteil 4 umgibt. Das Griffstück 8 ist relativ zu dem Schiebeteil 4 und/oder dem Aufnahmeteil 6 entlang einer Längsachse L des Schiebeteils 4 ausgebildet. Das heißt, das Griffstück 8 kann translatorisch entlang des Schiebeteils in die Verschieberichtung T verschoben oder bewegt werden. Um das Verschieben zu ermöglichen, umfassen der Aufnahmeteil 6 und das Griffstück 8 eine nicht näher dargestellte Öffnung, zum Aufnehmen und Führen des Schiebeteils 6 entlang der Verschieberichtung T.

Um die Eindringtiefe oder Einführtiefe zu quantifizieren, ist das Schiebeteil 4 vorliegend als Skalenkolben ausgebildet. Das heißt, auf dem Schiebeteil 4 ist eine Skala 4b angegeben, mit Messwerten oder Zahlenwerten, die die Eindringtiefe vorgeben. Im vorliegenden Ausführungsbeispiel gibt die Skala 4b zum Beispiel Zahlenwerte bis 4 Zentimeter an und ist in Millimeterschritte unterteilt. Somit kann die Eindringtiefe besonders präzise eingestellt werden.

Um die Eindringtiefe festzulegen, umfasst das Griffstück 8 einen Blockiermechanismus. Durch den Blockiermechanismus kann das Griffstück 8 an einer gewünschten Stelle entlang der Längsachse L gegen das Verschieben entlang der Verschieberichtung blockiert werden. In dieser Stellung bildet das Griffstück 8 einen Anschlag für das Schiebeteil 4 und/oder das Aufnahmeteil 6, insbesondere einen nicht näher dargestellten Kopf des Schiebeteils 4 und begrenzt dadurch die Eindringtiefe.

Der für die Blockierung verwendete Blockiermechanismus ist an sich aus dem Stand der Technik, insbesondere der EP 2 531 122 B1 bekannt. Im Folgenden ist der Blockiermechanismus anhand eines bevorzugten Ausführungsbeispiels näher beschrieben. Natürlich können aber auch andere bekannte Arten des Blockiermechanismus eingesetzt werden. Zur Erläuterung des Blockiermechanismus des Griffstücks 8 ist das Griffstück 8 in Fig. 1 in einer Explosionsdarstellung dargestellt.

Um die Blockierung zu realisieren, umfasst das Griffstück 8 ein Einstellelement 10, welches im Folgenden auch als Twistlock bezeichnet wird. Das Einstellelement 10 ist zum Verdrehen quer zur Längsachse L des Schiebeteils 4 ausgebildet. Das heißt das Einstellelement kann um das Schiebeteil herum in die eingezeichnete Verdrehrichtung R, also rotatorisch bewegt oder verdreht werden. Wie in Fig. 1 gezeigt ist das Einstellelement 10 vorliegend beispielhaft zweiteilig ausgebildet. Die beiden Teile der Einstelleinrichtung 10 können über vorbestimmte Verbindungselemente formschlüssig miteinander verbunden werden. Durch die zweiteilige Form, ergibt sich der Vorteil, dass das Einstellelement besonders einfach zusammengebaut oder zusammengesetzt werden kann.

Des Weiteren umfasst das Griffstück 8 zumindest ein, vorliegend beispielhaft zwei Wippelemente 12. Die Wippelemente 12 sind entlang der Längsachse L an gegenüberliegenden Seiten des Schiebeteils 2 zwischen dem Einstellelement 10 und dem Schiebeteil 4 angeordnet. Die Wippelemente sind zwar gemeinsam mit dem Einstellelement 10 entlang der Längsachse verschiebbar. Im Gegensatz zu dem Einstellelement 10 sind die Wippelemente jedoch radial oder drehbar ortsfest, also drehfest oder verdrehsicher, mit dem Schiebteil 4 verbunden.

Dazu umfasst das Schiebeteil 4 entlang der Längsachse L eine jeweilige Lagernut 4c und die Wippelemente umfassen einen jeweiligen Lagerzapfen 12c. Mit dem Lagerzapfen 12c wird das jeweilige Wippelement 12 in der zugeordneten Lagernuten 4c befestigt oder gelagert. Dadurch ist das Griffstück 8 über die Wippelemente 12 mit dem Schiebeteil gekoppelt oder verbunden. Die jeweilige Lagernut 4c gibt die Verschieberichtung T für das Griffstück 8 vor.

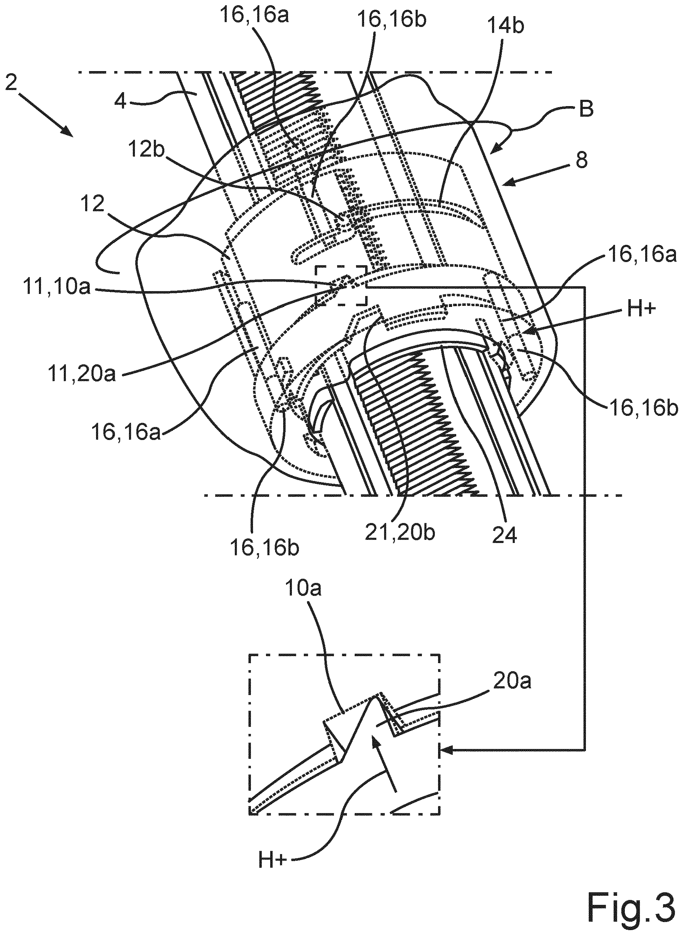

Das Einstellelement 10 bildet mit dem jeweiligen Wippelement 12 eine an sich bekannte Klemmeinrichtung aus. Dabei ist das Einstellelement 10 dazu ausgebildet, durch Verdrehen in eine Blockierrichtung B (vorliegend eine Rechtsdrehung), die quer zur Längsachse verläuft, in eine Blockierposition, das jeweilige Wippelement 12 in eine Arretierstellung zu dem Schiebeteil 4 zu verschwenken. In der Arretierstellung ist das Verschieben des Griffstücks 8 entlang der Längsachse blockiert. Das Griffstück ist somit arretiert und befindet sich in einem Nullzustand. Fig. 3 zeigt das Griffstück beispielhaft in dem Nullzustand.

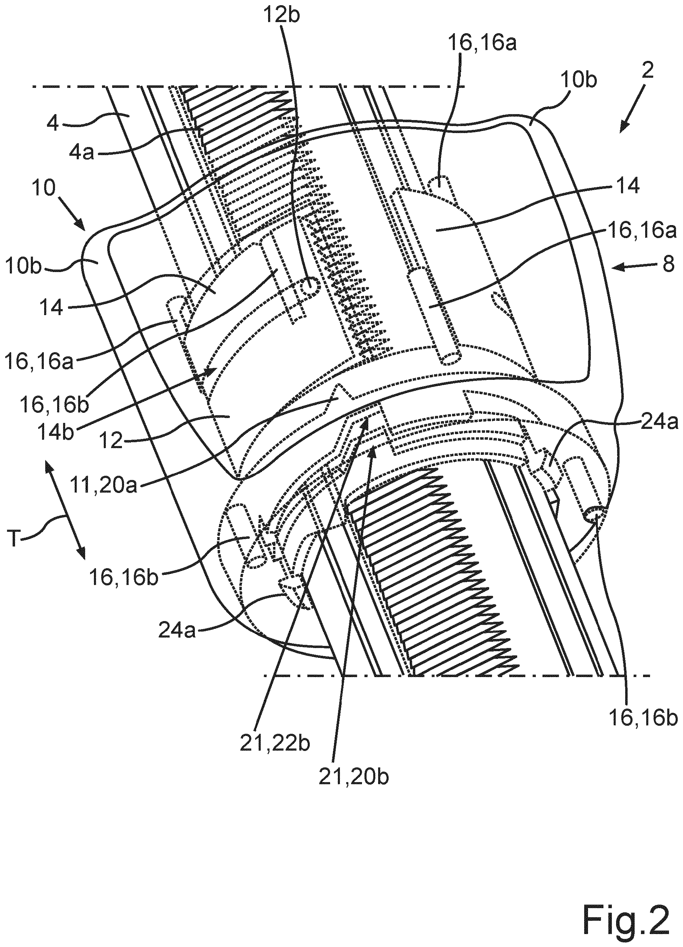

Im Gegensatz dazu ist das Einstellelement 10 weiterhin dazu ausgebildet, durch Verdrehen in eine von der Blockierrichtung B verschiedene Verstellrichtung V quer zur Längsachse L (vorliegend eine Linksdrehung) in eine von der Blockierposition verschiedene Verstellposition, das jeweilige Wippelement 12 in eine Freistellung zu dem Schiebeteil 4 zu verschwenken. In der Freistellung ist Verschieben des Griffstücks 8 entlang der Längsachse L freigegeben, also nicht mehr blockiert. Das Griffstück befindet sich in einem Ausgangszustand, wie er beispielhaft in Fig. 2 gezeigt ist.

Wie das jeweilige Wippelement 12 und das Einstellelement 10 ausgebildet sein können, um die Blockierung oder Arretierung zu ermöglichen ist an sich bekannt. Im folgenden Ausführungsbeispiel umfasst das jeweilige Wippelement an einer dem Schiebeteil 4 zugewandten Innenseite oder Auflagefläche eines Zahneinrichtung 12a. Das Schiebeteil 6 umfasst einer dem jeweiligen Wippelement 12 zugewandten Außenseite oder Auflagefläche ebenfalls eine korrespondierende Zahneinrichtung 4a.

Beim Verdrehen des Einstellelements 10 in die Blockierposition verkippt oder verschwenkt das Einstellelement 10 das jeweilige Wippelement 12 entlang der durch den jeweiligen Lagerzapfen beziehungsweise die jeweilige Lagernut 12c, 4c gebildete Achse, wodurch die Zahneinrichtungen 4a, 12a in Kontakt oder Verbindung miteinander gebracht werden. In der Arretierstellung liegen die Zahneinrichtungen 4a, 12a somit aneinander an. Dabei greifen Zahnelemente in Zahnelementaufnahmen der Zahneinrichtungen 4a, 12a ein. Es bildet sich eine formschlüssige Verbindung.

Beim Verdrehen in die Verstellposition des Einstellelements 10 wird das jeweilige Wippelement 12 hingegen so verschwenkt oder verkippt, dass der Kontakt zwischen den Zahneinrichtungen 4a, 12a gelöst wird. Das jeweilige Wippelement 12 befindet sich in der Freistellung. Die Blockierung ist gelöst.

Anstelle der Zahneinrichtungen 4a, 12a kann der Blockiermechanismus zum Beispiel durch einen Bremsbelag umgesetzt sein. In der Arretierstellung kann die jeweilige Auflagefläche des Wippelements 12 somit wie ein Bremskeil wirken.

Die beschriebene Vorrichtung 2 hat den Vorteil, dass sie besonders einfach einhändig bedient werden kann. Somit eignet sich die Vorrichtung 2 zum Beispiel in an sich bekannter Weise für den Einsatz in einem medizinischen Handstück. Ein entsprechendes medizinisches Handstück kann zum Beispiel für endoskopische Eingriffe eingesetzt werden. Dabei wird eine oder mehrere, insbesondere zwei der beschriebenen Vorrichtungen in bekannter Weise miteinander zusammengesetzt oder verbaut. Das resultierende Handstück kann mit einem oder mehreren medizinischen Geräten wie zum Beispiel einem Endoskop und/oder einer Absaugvorrichtung und/oder einer Aspirationsnadel über vorgesehene Schnittstellen verbunden werden. Mittels des Handstücks kann durch Einstellen der Eindringtiefe der jeweiligen Vorrichtung bei einem endoskopischen Eingriff zum Beispiel ein Überstand eines Aspirationsnadelteils der Aspirationsnadel aus einem Tubus, der beispielsweise durch das medizinische Handstück hindurchgeführt ist, festgelegt werden. Somit kann zum Beispiel eine Punktionstiefe für einen Tumor oder ein anderes Gewebestück besonders genau eingestellt sein. Ein medizinisches Handstück der bezeichneten Art ist an sich aus dem Stand der Technik bekannt und wird deshalb im Folgenden nicht näher beschrieben.

Um für das Einstellelement 10 eine jeweilige Bewegungsrichtung vorzugeben umfasst das Griffstück 8 zumindest eine, vorliegend beispielsweise zwei Führungsstrukturen 15. Die jeweilige Führungsstruktur 15 umfasst einen ersten Führungskörper und einen zweiten Führungskörper. Der erste Führungskörper ist vorliegend beispielhaft als Führungselement 14 ausgebildet. Das jeweilige Führungselement 14 ist vorliegend zum Beispiel an einer dem jeweiligen Wippelement 12 zugewandten Innenseite des Einstellelements 10 angebracht oder befestigt. Jedes der Führungselemente 14 umfasst einen Führungskanal 14b oder eine Führungsnut durch die ein diskreter Führungsverlauf für das Einstellelement 10 vorgegeben ist. Die Führungsschiene 14b ist vorliegend zum Beispiel als Ausschnitt oder Aussparung in dem Führungselement 14 ausgebildet.

Der jeweilige zweite Führungskörper ist vorliegend beispielhaft als Führungsstift 12b ausgebildet. Der Führungsstift 12b ist von dem jeweiligen Wippelement 12 umfasst. Der Führungsstift 12b ist an einer dem Einstellelement 10 zugewandten Außenseite des jeweiligen Wippelements 12 befestigt. Vorliegend bildet der Führungsstift 12b eine Erhebung oder eine Nase aus, die sich radial, also senkrecht zur Längsachse L in Richtung des Führungselements 14 erstreckt.

Der Führungsstift 12b und der Führungskanal 14b sind formschlüssig verbindbar. Dazu kann der Führungsstift 12b in die Führungsschiene 14b eingreifen. Das heißt der Führungsstift 12b ist in der Führungsschiene 14b anordenbar. Im angeordneten Zustand sind der Führungsstift 12b und die das Führungselement 14 relativ zueinander bewegbar, insbesondere in die Verdrehrichtung R und/oder die Verschieberichtung T. Die Bewegungsrichtung ist durch den Verlauf des Führungskanals 14b bestimmt. Dadurch, dass das Einstellelement 10 mit dem Führungselement 14 fest verbunden ist, ist somit auch das Einstellelement 10 im angeordneten Zustand relativ zu dem Wippelement 12 nur entlang des durch die Führungsschiene 14b vorgegebenen Führungsverlaufs bewegbar.

Der Führungsverlauf weist im vorliegenden Ausführungsbeispiel eine Stufenform oder Z-Form auf (siehe Fig. 2 oder Fig. 3). Das heißt, der Führungsverlauf weist drei verbundene Führungsverlaufsabschnitte auf. Zwei der Abschnitte verlaufen entlang der Verdrehrichtung R und sind durch einen Abschnitt verbunden, der in die Verschieberichtung T verläuft. Es ergibt sich ein Versatz des Führungsverlaufs in Längsrichtung L, vorliegend relativ zur Blockierrichtung B zum Beispiel nach unten. Der Versatz befindet sich in der Blockierposition des Einstellelements 10. Das heißt, die zur Verdrehrichtung R parallelen Abschnitte enden oder beginnen in der Blockierposition.

Die Blockierung zum Festlegen der Eindringtiefe kann sich in bestimmten Situationen ungewollt lösen. Um ein solchen ungewolltes Lösen der Blockierung zu vermeiden, ist zusätzlich zu dem Blockiermechanismus ein Fixiermechanismus vorgesehen. Durch den Fixiermechanismus kann die Blockierung fixiert oder verriegelt werden. Dann ist zum Beispiel ein zusätzlicher Kraftaufwand notwendig, um die Blockierung lösen zu können. Mit Lösen ist vorliegend insbesondere gemeint, dass das Einstellelement aus der Blockierposition in die Verstellposition und somit das jeweilige Wippelement 12 aus der Arretierstellung in die Freistellung bewegt wird.

Um den Fixiermechanismus umzusetzen, umfasst das Griffstück 8 eine Fixiereinrichtung 16. Vorliegend sind beispielswiese mehrere solcher Fixiereinrichtungen 16, insbesondere vier solcher Fixiereinrichtungen 16 vorgesehen. Die jeweilige Fixiereinrichtung 16 umfasst ein jeweiliges erstes Fixierelement 16a. Das jeweilige erste Fixierelement 16a ist mittels des Einstellelements 10 zu dem Schiebeteil 4 quer zur Längsachse L, also in die Verdrehrichtung R verdrehbar. Im vorliegenden Ausführungsbeispiel ist das jeweilige erste Fixierelement 16a dazu von dem Einstellelement 10 umfasst. Die vier ersten Fixierelemente 16a sind in der Verdrehrichtung R gleichverteilt in dem Einstellelement 10 angebracht. In Verschieberichtung T sind zwei der Fixierelemente 16a dabei jedoch nach oben, also in Richtung des Schiebeteils 4 versetzt angeordnet. Die übrigen beiden Fixierelemente 16a sind nach unten, also in Richtung des Aufnahmeteils 6 versetzt angeordnet.

Des Weiteren umfasst die jeweilige Fixiereinrichtung 16 ein zweites Fixierelement 16b das zu dem Schiebeteil 4 ortsfest oder drehfest angeordnet ist. Dazu kann das jeweilige zweite Fixierelement 16b zum Beispiel in das Schiebeteil 4 selbst integriert sein. Alternativ kann das jeweilige zweite Fixierelement 16, wie in dem Ausführungsbeispiel gezeigt, in eine zu dem Schiebeteil 6 drehfeste Komponente des Griffstücks 8 eingebaut sein. Vorliegend sind zwei der zweiten Fixierelemente 16b beispielhaft von jeweils einem der Wippelemente 12 umfasst. Die übrigen zwei Fixierelemente 16b sind beispielsweise von einem Entriegelungselement 20 einer im später noch näher bezeichneten Entriegelungseinrichtung 18 integriert oder angeordnet. Die zweiten Fixierelemente 16b sind analog zu den ersten Fixierelementen 16a relativ zur Verdrehrichtung R und Verschieberichtung T versetzt angeordnet.

In der Blockierposition des Einstellelements 10 sind das jeweilige erste und zweite Fixierelement 16a, 16b nun dazu ausgebildet, eine Haltekraft H zum Fixieren des Einstellelements gegen das Verdrehen quer zur Längsachse L auszuüben. Das heißt in der Blockierposition ist das Griffstück 8 blockiert und zusätzlich fixiert oder verriegelt. Durch die Haltekraft kann das Einstellelement 10 somit zunächst nicht mehr in die Verdrehrichtung R verdreht werden. Um das Verdrehen zu ermöglichen muss zunächst die Haltekraft H überwunden werden. Zum Überwinden der Haltekraft H ist bevorzugt ein größerer Kraftaufwand notwendig, als es zum Lösen der Blockierung an sich notwendig wäre.

In dem Ausführungsbeispiel in Fig. 1 ist die jeweilige Fixiereinrichtung 16 als passive Fixierung oder Verriegelung realisiert. Das heißt, das jeweilige erste Fixierelement 16a ist mittels des Einstellelements 10 durch dessen Verdrehen quer zur Längsachse in die Blockierposition relativ zu dem zweiten Fixierelement 16b bewegbar in eine vorgegebene Fixierstellung, in welcher die Haltekraft H maximal ist. Somit wird durch das Verdrehen des Einstellelements automatisch die Fixierung realisiert.

Das erste und zweite Fixierelement 16a, 16b sind vorliegend als Permanentmagneten ausgebildet. Dabei weisen das jeweilige erste und zweite Fixierelement 16a, 16b ein magnetisches Material mit einem gegenpoligen Magnetfeld auf. Die Haltekraft H ist somit durch die Magnetkraft bereitgestellt, mittels welcher sich die Permanentmagneten anziehen.

Vorliegend weisen die Permanentmagneten eine Zylinderform auf. Die Struktur der Magneten ist dabei so gewählt, dass deren Magnetkraft wirkt in Verschieberichtung T wirkt. Das heißt, die Haltekraft H ist vorliegend genau dann maximal, wenn ein jeweiliges erstes Fixierelement 16a parallel in einer Gerade zu dem zugeordneten zweiten Fixierelement 16b angeordnet ist (siehe zum Beispiel Fig. 3) Der Versatz der Fixierelemente 16a, 16b in Verdrehrichtung R ist dadurch vorliegend so gewählt, dass die zylinderförmigen Magnete eines ersten und des zugeordneten zweiten Fixierelements 16a, 16b in der Blockierposition mit den gegenpoligen Enden aneinander anliegen, vorzugsweise direkt miteinander in Berührung stehen. Es ergibt sich die vorgenannte Fixierstellung. Außerhalb der Blockierposition, insbesondere in der Verstellposition, sind die zugehörigen Fixierelemente 16a, 16b hingegen einerseits in Verschieberichtung T und andererseits in Verstellrichtung R zueinander versetzt oder beabstandet. Diese Stellung der Fixierelemente 16a, 16b wird im Folgenden auch als Entriegelungsstellung bezeichnet.

Alternativ zu dem Ausführungsbeispiel in Fig. 1 können die Fixierelemente 16a, 16b, zum Beispiel als Wälzlager mit zumindest einem Wälzkörper und einer Ausnehmung zum Aufnehmen des Wälzkörpers in der Fixierstellung ausgebildet sein. Alternativ ist beispielsweise natürlich auch eine aktive Fixierung denkbar. Dabei können die Fixierelemente 16a, 16b zum Beispiel als Sperrkörper und Ausnehmung zum Aufnehmen des Sperrkörpers ausgebildet sein. Zum Bewegen in die Fixierstellung ist eine zusätzliche zu dem Verdrehen vorgesehene Bedienhandlung einer Bedienperson notwendig.

Analog zu der Fixierung kann eine aktive oder passive Entriegelung, also ein passives oder aktives Lösen der Fixierung vorgesehen sein. Eine passive Entriegelung kann zum Beispiel dadurch realisiert sein, dass das Einstellelement 10 aus der Blockierposition heraus in die Verstellrichtung V verdreht wird. Erfolgt das Verdrehen mit einem genügend großen Kraftaufwand, kann die Haltekraft H der Fixiereinrichtung 16 gelöst werden. Dabei können die Fixierelemente 16a, 16b in die Entriegelungsstellung bewegt werden, in der die Haltekraft H zumindest reduziert insbesondere minimal oder nullifiziert ist. Durch das Verdrehen in die Verstellrichtung wird aber zusätzlich auch die Blockierung des jeweiligen Wippelements 12 gelöst, da das jeweilige Wippelement 12 von der Arretierstellung in die Freistellung bewegt wird.

Um die Fixierung noch sicherer zu machen, und eine ganz bewusste Entriegelung vorzusehen, kann die aktive Entriegelung vorgesehen sein. Dazu umfasst das Griffstück 8 einen Entriegelungsmechanismus, wie er nachfolgend näher beschrieben ist. Der Entriegelungsmechanismus ist im vorliegenden Ausführungsbeispiel mittels einer Entriegelungseinrichtung 18 des Griffstücks 8 und dem stufenförmigen Führungsverlauf der Führungsstruktur 15 umgesetzt.

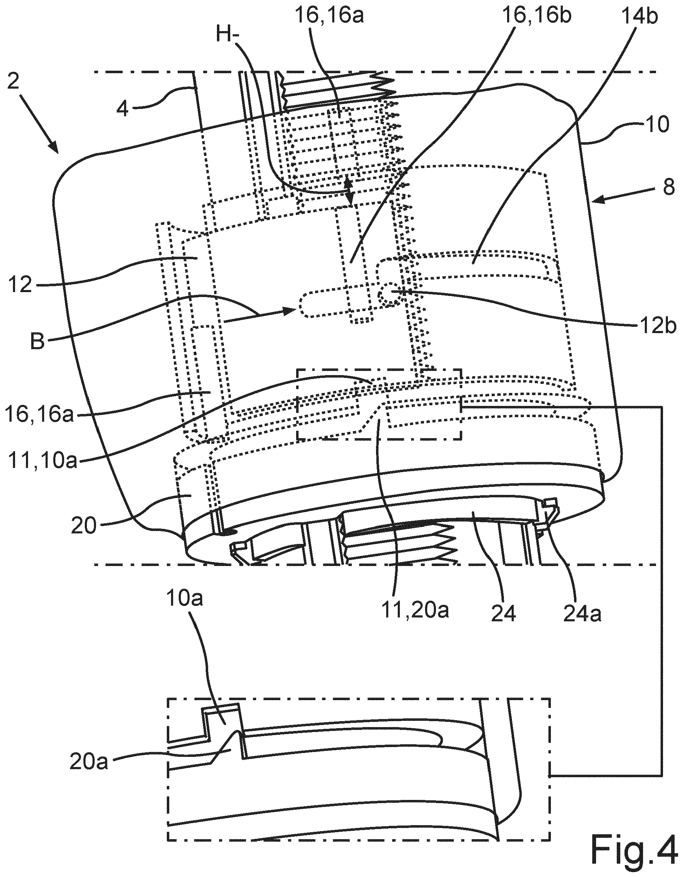

Wie in Fig. 4 gezeigt, ist zum Einleiten des Entriegelns zunächst ein Verschieben oder Ziehen des Einstellelements 10 entlang der Längsachse L, vorliegend insbesondere nach oben, vorgesehen, um die Magnetkraft der Fixierelemente 16a, 16b zu lösen. Die Bewegungsrichtung und der Bewegungsspielraum ist durch den zur Längsachse L parallel verlaufenden Abschnitt der Führungsschiene 14b vorgegeben.

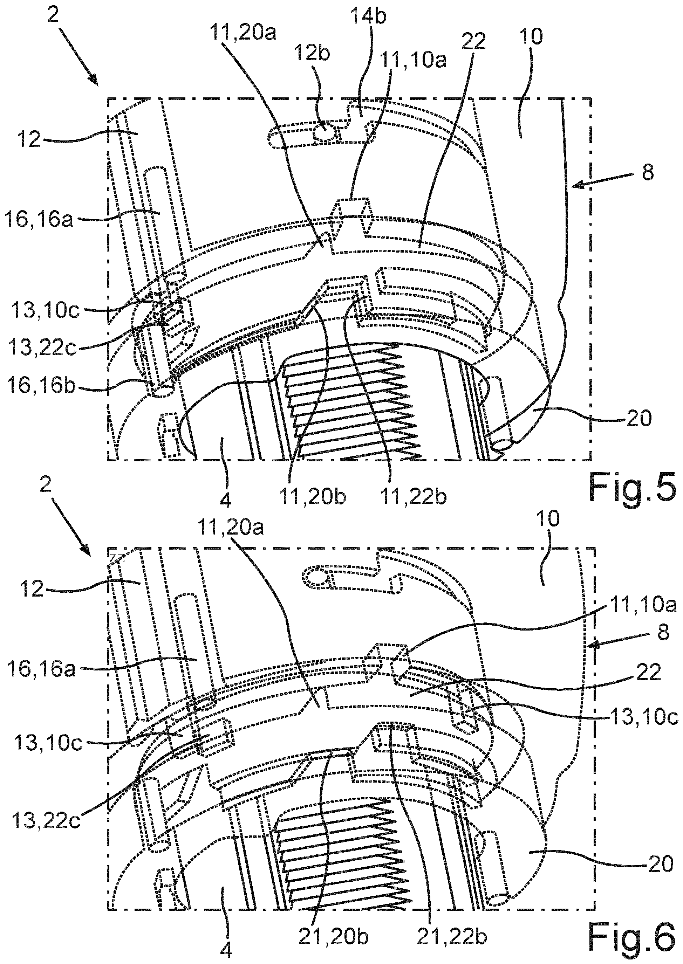

Anschließend ist ein Verdrehen des Einstellelements 10 weiter in die Blockierrichtung B vorgesehen, um die Entriegelung durchzuführen (siehe Fig. 5 und 6). Entsprechend kann gemäß dem Entriegelungsmechanismus das Einstellelement 10 zum Lösen der Fixierung dazu ausgebildet sein, durch Verdrehen aus der Blockierposition in die Blockierrichtung B eine von der Blockierposition und der Verstellposition verschiedene Entriegelungsposition einzunehmen. Fig. 6 zeigt das Griffstück 8 in einem Entriegelungszustand, in der sich das Einstellelement 10 in der Entriegelungsposition befindet.

In der Entriegelungsposition sind das erste und zweite Fixierelement 16a, 16b in die vorgenannte Entriegelungsstellung bewegt. Das jeweilige Wippelement 12 behält dabei in der Entriegelungsposition des Einstellelements 10 die Arretierstellung bei. Das heißt, in der Entriegelungsposition ist das Griffstück 8 blockiert aber nicht mehr fixiert oder verriegelt.

Um die Entriegelungsstellung der Fixierelemente 16a, 16b bei dem Verdrehen in die Entriegelungsposition oder zurück in die Verstellposition zu halten, ist die Entriegelungseinrichtung 18 vorgesehen. Die Entriegelungseinrichtung 18 ist beispielsweise dreiteilig aufgebaut und zwischen dem Schiebeteil 4 und dem Einstellelement 10 angeordnet. In den Ausführungsbeispielen ist die Entriegelungseinrichtung entlang der Längsachse unterhalb des jeweiligen Wippelements 12 angeordnet.

Die Entriegelungseinrichtung 18 umfasst ein erstes Einstellelement 20, ein zweites Einstellelement 22 und ein Signalisierungselement 24. Das erste Entriegelungselement 20 ist zwischen dem Einstellelement 10 und dem Schiebeteil 6 angeordnet. Das zweite Einstellelement 22 ist entlang der Verschieberichtung T zumindest bereichsweise oder abschnittsweise von dem ersten Entriegelungselement 20 überlappt oder umgeben und zwischen dem Schiebeteil 4 und dem Einstellelement 10 angeordnet. Das Signalisierungselement 24 ist zwischen dem Schiebeteil 6 und dem ersten und zweiten Entriegelungselement 20, 22 angeordnet.

Das erste Einstellelement 20 ist relativ zu dem Schiebeteil 6 drehfest angeordnet. Allerdings ist das Einstellelement 10 relativ zu dem erste Entriegelungselement 20 entlang der Längsachse L verschiebbar. Das zweite Entriegelungselement 22 ist relativ zu dem Einstellelement 10 gegen das Verschieben entlang der Längsachse L ortsfest. Allerdings ist das zweite Entriegelungselement 22 relativ zu dem Einstellelement 10 in die Verdrehrichtung R verdrehbar, insbesondere zumindest abschnittweise verdrehbar. Das Signalisierungselement 24 ist hingegen vollständig ortsfest oder drehfest zu dem Einstellelement 10 ausgebildet. Das erste Einstellelement 20 ist vorzugweise relativ zu dem Signalisierungselement 24 entlang der Längsachse L verschiebbar.

Das erste Entriegelungselement 21 und das Einstellelement 10 bilden zumindest eine, vorliegend beispielsweise zwei Abstandshaltestrukturen 11 zueinander aus. Beim Verdrehen des Einstellelements 10 quer zur Längsachse außerhalb der Blockierposition sind das Einstellelement 10 und das erste Entriegelungselement 21 mittels der jeweiligen Abstandshaltestruktur 11 relativ zueinander in einem vorgegebenen Abstand gehalten. Das heißt das erste Entriegelungselement 20 und das Einstellelement 10 sind außerhalb der Blockierposition gegeneinander abgestützt. Dadurch wird die Entriegelungsstellung der Fixierelemente 16a, 16b realisiert (siehe zum Beispiel Fig. 5 und 6).

Die jeweilige Abstandshaltestruktur 11 ist vorliegend durch ein Abstützteil 20a und ein Halteteil 10a als Abstandshaltekörper gebildet. Das Abstützteil 20a erstreckt sich als Vorsprung oder Nase entlang der Längsachse L aus dem ersten Entriegelungselement 20 heraus. Das Halteteil 10a ist von dem Einstellelement 10 umfasst und durch eine Auflagefläche oder Abstützfläche gebildet. Die Auflagefläche verläuft axial entlang der dem Schiebeteil 4 zugewandten Innenseite des Einstellelements 10 entlang der Verdrehrichtung R. Zum Realisieren der Entriegelungsstellung liegt das Abstützteil 20a auf der Auflagefläche des Halteteils 10a auf. Beim Verdrehen gleitet die Nase somit entlang der Auflagefläche.

Um die Fixierung noch zu verbessern, umfasst die Abstandshaltestruktur 11 einen Verriegelungsmechanismus. Mittels diesem sind das erste Entriegelungselement 20 und das Einstellelement 10 in der Blockierposition formschlüssig verbindbar, wodurch der vorgegebene Abstand reduziert und die Fixierstellung der Fixierelemente 16a, 16b umgesetzt ist. Vorliegend umfasst das beschriebene Halteteil 10a eine zu dem Vorsprung des Abstützteils 20a korrespondierende Aufnahme oder Aussparung. Diese Aussparung erstreckt sich entlang der Verschieberichtung T korrespondierend zu dem Abstützteil 20a. In der Blockierposition ist die Aufnahme ausgebildet die Nase des Abstützteils 20a aufzunehmen. Die Nase kann in die Aufnahme einrasten oder eingreifen. Dadurch wird der durch die Nase vorgegebene Abstand reduziert und die Fixierelemente 16a, 16b können in die Fixierstellung gebracht werden.

Das erste Entriegelungselement 20 ist über einen Führungszapfen 20c mit einer Führungsnut 24c mit dem Signalisierungselement 24 gekoppelt oder verbunden. Die Führungsnut 24c erstreckt sich in der Verschieberichtung T entlang des Signalisierungselements 24. Somit ist das erste Entriegelungselement 20 relativ zu dem Signalisierungselement 24 entlang der Längsrichtung L verschiebbar.

In dem aufgenommenen Zustand begrenzt die Führungsnut 24c die Bewegung des ersten Entriegelungselements 20 vorliegend nach oben. Dadurch kann durch das Ziehen an dem Einstellelement 10 zum Lösen der Fixierung kann die Nase zumindest bereichsweise, vorzugsweise vollständig aus der Aufnahme herausgeführt werden (siehe Fig. 4). Dabei löst sich die Haltekraft der Fixierelemente 16a, 16b zumindest so weit, dass die Magnetkraft nicht mehr stark genug ist, um das erste Entriegelungselement 20 zu halten. Das erste Entriegelungselement 20 fällt dadurch in Richtung der Schwerkraft vorliegend nach unten in Richtung des Aufnahmeteils 6.

In Richtung des Aufnahmeteils 6 der Vorrichtung 2 umfasst das Signalisierungselement 24 zumindest einen, vorliegend beispielsweise zwei, insbesondere beispielsweise drei oder mehr Anschläge 24a. Mittels des jeweiligen Anschlags 24a ist von einer Seite zumindest in der Entriegelungsstellung, also außerhalb der Blockierposition das erste Entriegelungselement 20 abgestützt. Auf der gegenüberliegenden Seite kann zum Beispiel das Aufnahmeteil 6 mittels des Anschlags 24a gegenüber dem Griffteil 8 abgestützt sein.

Da das Signalisierungselement zu dem Einstellelement 10 und dem ersten Entriegelungselement 20 ortfest ausgebildet ist, kann in der Blockierposition das Signalisierungselement 24 entlang der Längsachse L (vorliegend beispielsweise nach unten) über das Einstellelement 10 hinausragen. Das Signalisierungselement 24 ist bereichsweise freigelegt und somit durch die Bedienperson sichtbar. Dadurch kann das Blockieren und insbesondere auch das Fixieren des Griffteils 8 mittels des Signalisierungselements 24 signalisiert werden (siehe Fig. 3). Insbesondere kann das Signalisierungselement 24 zum Beispiel in einer Signalfarbe, wie zum Beispiel rot, eingefärbt sein.

Das zweite Entriegelungselement 22 bildet mit dem ersten Entriegelungselement 20 zumindest eine, vorliegend beispielsweise zwei Verschiebestrukturen 21 aus. Mittels der jeweiligen Verschiebestruktur 21 sind das Einstellelement 10 und das erste Entriegelungselement 20 entlang der Längsachse L relativ zueinander in den vorgegebenen Abstand verschiebbar. Das heißt, die Entriegelungsstruktur 21 wird dazu eingesetzt, die Entriegelung automatisch durch das Verdrehen des Einstellelements 10 in die Entriegelungsposition zu realisieren. Vorzugsweise kann die Verschiebestruktur 21 auch zum Halten des vorgenannten Abstands analog zu der Abstandshaltestruktur 11 eingesetzt werden.

Die jeweilige Verschiebestruktur 21 ist vorliegend beispielsweise durch zwei Verschiebekörper, nämlich einen ersten Verschiebekörper 20b und einen zweiten Verschiebekörper 22b gebildet. Der erste Verschiebekörper 20b ist an der dem zweiten Entriegelungselement 22 zugewandten Innenseite des ersten Entriegelungselements 20 angeordnet. Der erste Verschiebekörper 20b ist beispielsweise als Ausbuchtung oder Vorsprung ausgebildet, der sich entlang der Längsrichtung L (vorliegend beispielsweise nach oben) zu dem zweiten Entriegelungselement 22 hin erstreckt. Der erste Verschiebekörper 20b weist eine Auflagefläche (vorliegend die Oberseite) auf, mittels welcher der erste Verschiebekörper 20b gegen den zweiten Verschiebekörper 22b abgestützt sein kann.

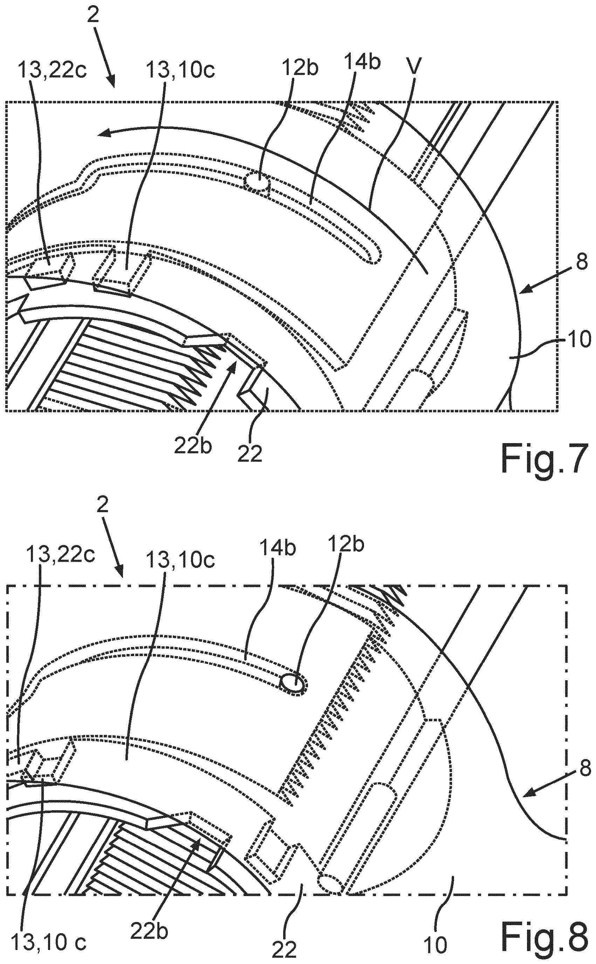

Der zweite Verschiebekörper 22b ist von dem zweiten Entriegelungselement 22 umfasst. Der zweite Verschiebekörper 22b umfasst eine Auflagefläche oder Abstützfläche für die Auflagefläche des ersten Verschiebekörpers 20b. Wie in Fig. 7 und 8 gezeigt, ist die Auflagefläche des zweiten Verschiebekörpers 22b durch einen unteren Randbereich des zweiten Entriegelungselements 22 gebildet.

Der zweite Verschiebekörper 22b umfasst zudem eine zu dem ersten Verschiebekörper 20b, insbesondere der Ausbuchtung korrespondierende Aufnahme oder Aussparung. In diese Aufnahme oder Aussparung kann der erste Verschiebekörper 20b in der Blockierposition aufgenommen sein, wie in der Fig. 3 gezeigt. Dadurch bilden der erste Verschiebekörper 20b und der zweite Verschiebekörper 22b eine formschlüssige Verbindung aus. Wie in Fig. 1 gezeigt weisen die Aussparung und der Vorsprung eine Rechteckform auf, bei der eine Seite schräg zur Verschieberichtung T verläuft. Das heißt, es ist eine Rampe gebildet. Durch diese Rampe kann beim Verdrehen des Einstellelements 10 aus der Blockierposition in die Blockierrichtung der Vorsprung entlang der Aussprung besonders einfach gleiten und so das zweite Entriegelungselement 22 und insbesondere das damit verbundene Einstellelement 10 und das erste Entriegelungselement 20 in den vorgegebenen Abstand zueinander gebracht werden (siehe Fig. 6 und 7). Wie in Fig. 1 gezeigt kann die Entriegelungseinrichtung mehrere, zum Beispiel zwei der zuvor beschriebenen Abstandshaltestrukturen und Verschiebestrukturen umfassen.

Schließlich bilden das zweite Entriegelungselement 22 und das Einstellelement 10 zumindest eine, vorliegend beispielsweise zwei Koppelstrukturen 13 zueinander aus. Mittels der jeweiligen Koppelstruktur 13 kann das zweite Entriegelungselement 22 zumindest abschnittsweise beim Verdrehen des Einstellelements 10 quer zur Längsachse L mitgeführt werden. Insbesondere wird das zweite Einstellelement 22 nur beim Verdrehen von der Blockierposition in die Entriegelungsposition und zumindest abschnittsweise beim Verdrehen des Einstellelements 10 von der Entriegelungsposition in die Verstellposition mitgeführt.

Die jeweilige Koppelstruktur 13 ist vorliegend beispielsweise durch zwei Mitnahmekörper 11c und einen jeweiligen Mitführkörper 22c gebildet. Der jeweilige Mitnahmekörper 11c ist als radial zur Längsachse L auf der dem zweiten Entriegelungselement 22 zugewandten Innenseite des Einstellelements 10 abragender Vorsprung oder Block gebildet. Der jeweilige Mitführkörper 22c ist als radial zur Längsachse L aus der in Richtung des Einstellelements 10 herausragenden Außenseite des zweiten Entriegelungselements 22 ausgebildet. Im gekoppelten Zustand beaufschlagt der jeweilige Mitnahmekörper 11c den Mitführkörper 22c mit einer durch das Verdrehen erzeugten Kraft, mittels welcher der Mitnahmekörper 22c mit dem Mitführkörper 11c rotatorisch mitgeführt wird.

Die beiden Mitführkörper 11c sind entlang der Verdrehrichtung R versetzt angeordnet. Der Mitnahmekörper 22c ist zwischen den beiden Mitführkörpern 11c angeordnet. Wie in Fig. 5 und 6 gezeigt, ist die axiale Anordnung der Mitführkörper 11c so gewählt, dass einer der Mitführkörper 11c in der Blockierposition mit dem Mitnahmekörper 22c gekoppelt ist. Beim Verdrehen des Einstellelements 10 in die Blockierrichtung wird dadurch das zweite Entriegelungselement 22 mit verdreht. Dadurch wird die Verschiebestruktur 21 aktiviert.

Fig. 7 und Fig. 8 zeigen die Rückführung des Einstellelements 10 von der Entriegelungsposition zurück in die Ausgangsposition. Fig. 7 zeigt das Griffstück 8 dazu in einem Zustand, in dem das Einstellelement 10 in etwa zu zwei Dritteln ausgehend von der Entriegelungsposition zurück in die Verstellposition verdreht ist. Zur besseren Übersicht ist von der Entriegelungseinrichtung 18 nur das zweite Einstellelement 22 dargestellt um die Funktion der Koppelstruktur 13 übersichtlicher darzustellen.

In Fig.7 befinden sich der andere der Mitführkörper 11c sowie der Mitnahmekörper 22c befinden sich gerade noch nicht in dem gekoppelten Zustand. Die Kopplung erfolgt aber noch vor Erreichen der Verstellposition. Somit wird das zweite Entriegelungselement 22 erst im letzten Abschnitt der Führungsschiene 14b beim Verdrehen in die Verstellrichtung V mitgeführt werden. Bezogen auf Fig. 6 bleibt somit durch die Verschiebestruktur 21 der Abstand zwischen dem ersten Entriegelungselement 20 und dem Einstellelement 10 erhalten, ohne dass das Abstützteil 20a und die Ausnahme des Halteteils 10a beim Verdrehen und Verschieben zurück in die Verstellposition ineinandergreifen. Erst wenn der Abstützkörper relativ zu der Aufnahme wieder versetzt ist, wird das zweite Einstellelement 22 durch die Koppelstruktur 13 mitgeführt, bis sich das Einstellelement 10 wieder in der Ausgangsstellung, also in der Verstellposition befindet.

Insgesamt zeigen die Ausführungsbeispiele, wie ein Lockable Twistlock, also ein verriegelbares Einstellelement 10, zum Beispiel für ein Griffstück 8 eines medizinischen Handstücks realisiert sein kann.

## Patentansprüche

1. Vorrichtung (2) zum Festlegen einer Eindringtiefe eines rohr- oder stangenförmigen Schiebeteils (4), insbesondere eines medizinischen Aspirationsnadelträger-Teils in einem Aufnahmeteil (6), mit einem ein Griffstück (8), welches das Schiebeteil (4) umgibt, und welches relativ zu dem Schiebeteil (4) zum Verschieben entlang einer Längsachse (L) des Schiebeteils (4) ausgebildet ist, und das Griffstück (8) umfassend:
- ein Einstellelement (10) welches zum Verdrehen quer zur Längsachse (L) des Schiebeteils (4) ausgebildet ist, und
- zumindest ein Wippelement (12), welches zwischen dem Einstellelement (10) und dem Schiebeteil (4) angeordnet ist, wobei
- das Einstellelement (10) dazu ausgebildet ist, durch Verdrehen in eine Blockierrichtung (B) quer zur Längsachse (L) in eine Blockierposition das jeweilige Wippelement (12) in eine Arretierstellung zu dem Schiebeteil (4) zu verschwenken, in der das Verschieben des Griffstücks (8) entlang der Längsachse (L) blockiert ist, und
- das Einstellelement (10) dazu ausgebildet ist, durch Verdrehen in eine von der Blockierrichtung (B) verschiedene Verstellrichtung (V) quer zur Längsachse (L) in eine von der Blockierposition verschiedenen Verstellposition das jeweilige Wippelement (12) in eine Freistellung zu dem Schiebeteil (4) zu verschwenken, in der das Verschieben des Griffstücks (8) entlang der Längsachse (L) freigegeben ist,
**gekennzeichnet durch**,
- zumindest eine Fixiereinrichtung (16) mit einem mittels des Einstellelements (10) zu dem Schiebeteil (4) quer zur Längsachse (L) verdrehbaren ersten Fixierelement (16a) und einem gegen das Verdrehen quer zur Längsachse (L) zu dem Schiebeteil (4) ortsfesten zweiten Fixierelement (16b), wobei
- das erste und zweite Fixierelement (16a, 16b) dazu ausgebildet sind, in der Blockierposition eine Haltekraft (H) zum Fixieren des Einstellelements (10) gegen das Verdrehen quer zur Längsachse (L) auszuüben, wobei
- das erste Fixierelement (16a) von dem Einstellelement (10) umfasst ist und zum Lösen der Fixierung die Haltekraft (H) zu überwinden ist.

2. Vorrichtung nach Anspruch 1, wobei das erste Fixierelement (16a) einen Sperrkörper ausbildet, und das zweite Fixierelement (16b) eine Ausnehmung zum Aufnehmen des Sperrkörpers ausbildet, wobei der Sperrkörper dazu ausgebildet ist, in der Blockierposition des Einstellelements (10) mittels einer Bedienhandlung einer Bedienperson in eine Fixierstellung bewegt zu werden, in welcher die Haltekraft (H) maximal ist.

3. Vorrichtung nach Anspruch 1, wobei das Einstellelement (10) dazu ausgebildet ist, durch das Verdrehen quer zur Längsachse (L) in die Blockierposition, das erste Fixierelement (16a) in eine Fixierstellung zu dem zweiten Fixierelement (16b) zu bewegen, in welcher die Haltekraft (H) maximal ist.

4. Vorrichtung nach Anspruch 3, wobei das erste Fixierelement (16a) und das zweite Fixierelement (16b), ein magnetisches Material umfassen und zueinander ein gegenpoliges Magnetfeld ausbilden.

5. Vorrichtung nach Anspruch 3, wobei das erste Fixierelement (16a) als Wälzlager mit zumindest einem Wälzkörper ausgebildet ist, und das zweite Fixierelement (16b) eine Ausnehmung zum Aufnehmen des Wälzkörpers in der Fixierstellung ausbildet.

6. Vorrichtung nach einem der vorhergehenden Ansprüche, wobei das Einstellelement (10) und das jeweilige Wippelement (12) eine Führungsstruktur (15) ausbilden, welche einen Führungsverlauf zumindest für das Verdrehen des Einstellelements (10) quer zur Längsachse (L) relativ zu dem jeweiligen Wippelement (12) vorgibt, wobei das Einstellelement (10) entlang des Führungsverlaufs in die jeweilige Position verstellbar ist.

7. Vorrichtung nach Anspruch 6, wobei die Führungsstruktur (15) einen der Blockierposition zugeordneten Führungsverlaufsabschnitt umfasst, welcher entlang der Längsachse (L) verläuft, und das Einstellelement (10) zum Lösen der Fixierung entlang des Führungsverlaufsabschnitts relativ zu dem jeweiligen Wippelement (12) verschiebbar ist.

8. Vorrichtung nach einem der Ansprüche 3 bis 7, wobei das Einstellelement (10) zum Lösen der Fixierung dazu ausgebildet ist, durch Verdrehen aus der Blockierposition in die Blockierrichtung (B) eine von der Blockierposition und der Verstellposition verschiedene Entriegelungsposition einzunehmen, und dadurch das erste und zweite Fixierelement (16a, 16b) in eine Entriegelungsstellung zu bewegen, in welcher die Haltekraft (H) im Vergleich zu der Fixierstellung reduziert ist, wobei das jeweilige Wippelement (12) in der Entriegelungsposition des Einstellelements (10) die Arretierstellung beibehält.

9. Vorrichtung nach Anspruch 8, wobei das Griffstück (8) eine Entriegelungseinrichtung (18) umfasst, welche zwischen dem Schiebteil (4) und dem Einstellelement (10) angeordnet ist, und die dazu ausgebildet ist, beim Verdrehen des Einstellelements (10) quer zur Längsachse die jeweiligen Fixierelemente (16a, 16b) in der Entriegelungsstellung zu halten.

10. Vorrichtung nach Anspruch 9, wobei die Entriegelungseinrichtung (18) ein erstes Entriegelungselement (20) umfasst, welches gegen das Verdrehen quer zur Längsachse zu dem Schiebeteil (4) ortsfest angeordnet ist, und das Einstellelement (10) und das erste Entriegelungselement (20) eine Abstandshaltestruktur (11) ausbilden, wobei die Abstandshaltestruktur (11) dazu ausgebildet ist, beim Verdrehen des Einstellelements (10) quer zur Längsachse (L) das Einstellelement (10) und das erste Entriegelungselement (20) entlang der Längsachse (L) relativ zueinander in einem vorgegebenen Abstand zueinander zu halten, und dadurch die Entriegelungsstellung der Fixierelemente (16a, 16b) zu realisieren, und wobei die Abstandhaltestruktur (11) einen Verbindungsmechanismus umfasst, mittels welchem das erste Entriegelungselement (20) und das Einstellelement (10) in der Blockierposition formschlüssig verbunden sind und dabei der vorgegebene Abstand reduziert ist.

11. Vorrichtung nach Anspruch 10, wobei die Entriegelungseinrichtung (18) ein Signalisierungselement (24) umfasst, welches durch Verschieben des ersten Entriegelungselements (20) nur in der Blockierposition zumindest bereichsweise entlang der Längsachse (L) über das Einstellelement (10) zum Signalisieren des Blockierens und Fixierens hinausragt.

12. Vorrichtung nach Anspruch 11, wobei die Entriegelungseinrichtung (18) ein zweites Entriegelungselement (22) umfasst, welches gegen das Verschieben entlang der Längsachse (L) zu dem Einstellelement (10) ortsfest angeordnet ist, wobei das erste und zweite Entriegelungselement (20, 22) eine Verschiebestruktur (21) ausbilden, wobei die Verschiebestruktur (21) dazu ausgebildet ist, beim Verdrehen des Einstellelements (10) quer zur Längsachse (L) aus der Blockierposition heraus in die Entriegelungsposition das Einstellelement (10) und das erste Entriegelungselement (20) entlang der Längsachse (L) relativ zueinander in den vorgegebenen Abstand zueinander zu verschieben.

13. Vorrichtung nach Anspruch 12, wobei das Einstellelement (10) und das zweite Entriegelungselement (22) eine Koppelstruktur (13) zum zumindest abschnittsweisen Mitführen des zweiten Entriegelungselements (22) beim Verdrehen des Einstellelements (10) quer zur Längsachse (L) ausbilden, wobei die Koppelstruktur (13) ausgebildet ist, die Abstandshaltestruktur (11) und die Verschiebestruktur (21) beim Verschieben des Einstellelements (10) aus der Entriegelungsposition in die Verstellposition quer zur Längsachse (L) zu versetzen und dadurch den vorgegebenen Abstand beim Verdrehen in die Verstellrichtung (V) zu halten.

14. Medizinisches Handstück für ein medizinisches Gerät, mit einer ersten und einer zweiten Vorrichtung (2) nach einem der vorhergehenden Ansprüche, wobei die erste Vorrichtung (2) ausgebildet ist, die Eindringtiefe eines ersten rohrförmigen Schiebeteiles (4) in einem ersten Aufnahmeteil (6) festzulegen, und wobei die zweite Vorrichtung (2) ausgebildet ist, die Eindringtiefe eines zweiten rohrförmigen Schiebeteiles (4) in einem zweiten Aufnahmeteil (6) festzulegen, und die erste Vorrichtung (2) und die zweite Vorrichtung (2) derart hintereinander angeordnet sind, dass das erste Schiebeteil (4) der ersten Vorrichtung (2) zumindest zum Teil als zweites Aufnahmeteil (6) für das zweite Schiebeteil (4) der zweiten Vorrichtung (2) ausgebildet ist, wobei das erste Aufnahmeteil (6) und das zweite Schiebeteil (4) eine jeweilige Verbindungsschnittstelle zum Verbinden von Elementen des medizinischen Geräts aufweisen.

## Claims

1. An apparatus (2) for setting a penetration depth of a tubular or rod-shaped sliding part (4), in particular of a medical aspiration needle carrier part in a receiving part (6), with a handle piece (8), which surrounds the sliding part (4), and which is formed for sliding along a longitudinal axis (L) of the sliding part (4) in relation to the sliding part (4), and the handle piece (8) comprising:
- an adjusting element (10), which is formed for rotating transversely to the longitudinal axis (L) of the sliding part (4), and
- at least one rocker element (12), which is arranged between the adjusting element (10) and the sliding part (4), wherein
- the adjusting element (10) is formed to pivot the respective rocker element (12) into an arresting pose to the sliding part (4), in which sliding of the handle piece (8) along the longitudinal axis (L) is blocked, by rotating into a blocking direction (B) transversely to the longitudinal axis (L) into a blocking position, and
- the adjusting element (10) is formed to pivot the respective rocker element (12) into a release pose to the sliding part (4), in which sliding of the handle piece (8) along the longitudinal axis (L) is released, by rotating into an adjusting direction (V) different from the blocking direction (B) transversely to the longitudinal axis (L) into an adjusting position different from the blocking position,
**characterized by**
- at least one fixing device (16) with a first fixing element (16a) rotatable transversely to the longitudinal axis (L) to the sliding part (4) by means of the adjusting element (10) and a second fixing element (16b) stationary to the sliding part (4) against the rotation transverse to the longitudinal axis (L), wherein
- the first and the second fixing element (16a, 16b) are formed to exert a retaining force (H) for fixing the adjusting element (10) against the rotation transverse to the longitudinal axis (L) in the blocking position, wherein
- the first fixing element (16a) is included by the adjusting element (10) and the retaining force (H) has to be overcome for releasing the fixing.

2. The apparatus according to claim 1, wherein the first fixing element (16a) forms a barrier body, and the second fixing element (16b) forms a notch for receiving the barrier body, wherein the barrier body is formed to be moved into a fixing pose, in which the retaining force (H) is maximum, by means of an operating action of an operator in the blocking position of the adjusting element (10).

3. The apparatus according to claim 1, wherein the adjusting element (10) is formed to move the first fixing element (16a) into a fixing pose to the second fixing element (16b), in which the retaining force (H) is maximum, by rotating transversely to the longitudinal axis (L) into the blocking position.

4. The apparatus according to claim 3, wherein the first fixing element (16a) and the second fixing element (16b) include a magnetic material and form a magnetic field of opposite polarity to each other.

5. The apparatus according to claim 3, wherein the first fixing element (16a) is formed as a rolling bearing with at least one rolling body, and the second fixing element (16b) forms a notch for receiving the rolling body in the fixing pose.

6. The apparatus according to any one of the preceding claims, wherein the adjusting element (10) and the respective rocker element (12) form a guide structure (15), which presets a guide course at least for rotating the adjusting element (10) transversely to the longitudinal axis (L) in relation to the respective rocker element (12), wherein the adjusting element (10) is adjustable into the respective position along the guide course.

7. The apparatus according to claim 6, wherein the guide structure (15) includes a guide course section associated with the blocking position, which extends along the longitudinal axis (L), and the adjusting element (10) is slidable along the guide course section in relation to the respective rocker element (12) for releasing the fixing.

8. The apparatus according to any one of claims 3 to 7, wherein the adjusting element (10) is formed to occupy an unlocking position different from the blocking position and the adjusting position by rotating from the blocking position into the blocking direction (B) for releasing the fixing, and thereby to move the first and the second fixing element (16a, 16b) into an unlocking pose, in which the retaining force (H) is reduced compared to the fixing pose, wherein the respective rocker element (12) maintains the arresting pose in the unlocking position of the adjusting element (10).

9. The apparatus according to claim 8, wherein the handle piece (8) includes an unlocking device (18), which is arranged between the sliding part (4) and the adjusting element (10), and which is formed to maintain the respective fixing elements (16a, 16b) in the unlocking pose upon rotating the adjusting element (10) transversely to the longitudinal axis.

10. The apparatus according to claim 9, wherein the unlocking device (18) includes a first unlocking element (20), which is stationarily arranged to the sliding part (4) against the rotation transverse to the longitudinal axis, and the adjusting element (10) and the first unlocking element (20) form a spacing structure (11), wherein the spacing structure (11) is formed to maintain the adjusting element (10) and the first unlocking element (20) at a preset distance in relation to each other along the longitudinal axis (L) upon rotating the adjusting element (10) transversely to the longitudinal axis (L), and thereby to realize the unlocking pose of the fixing elements (16a, 16b), and wherein the spacing structure (11) includes a connection mechanism, by means of which the first unlocking element (20) and the adjusting element (10) are connected in form-fit manner in the blocking position and therein the preset distance is reduced.

11. The apparatus according to claim 10, wherein the unlocking device (18) includes a signaling element (24), which protrudes beyond the adjusting element (10) at least in certain areas along the longitudinal axis (L) for signaling the blocking and fixing by sliding the first unlocking element (20) only in the blocking position.

12. The apparatus according to claim 11, wherein the unlocking device (18) includes a second unlocking element (22), which is stationarily arranged to the adjusting element (10) against sliding along the longitudinal axis (L), wherein the first and the second unlocking element (20, 22) form a sliding structure (21), wherein the sliding structure (21) is formed to slide the adjusting element (10) and the first unlocking element (20) into the preset distance to each other in relation to each other along the longitudinal axis (L) upon rotating the adjusting element (10) transversely to the longitudinal axis (L) out of the blocking position into the unlocking position.

13. The apparatus according to claim 12, wherein the adjusting element (10) and the second unlocking element (22) form a coupling structure (13) for carrying along the second unlocking element (22) at least in sections upon rotating the adjusting element (10) transversely to the longitudinal axis (L), wherein the coupling structure (13) is formed to offset the spacing structure (11) and the sliding structure (21) transversely to the longitudinal axis (L) upon sliding the adjusting element (10) from the unlocking position into the adjusting position and thereby to maintain the preset distance upon rotating into the adjusting direction (V).

14. A medical handpiece for a medical appliance, with a first and a second apparatus (2) according to any one of the preceding claims, wherein the first apparatus (2) is formed to set the penetration depth of a first tubular sliding part (4) in a first receiving part (6), and wherein the second apparatus (2) is formed to set the penetration depth of a second tubular sliding part (4) in a second receiving part (6), and the first apparatus (2) and the second apparatus (2) are arranged one behind the other such that the first sliding part (4) of the first apparatus (2) is at least partially formed as a second receiving part (6) for the second sliding part (4) of the second apparatus (2), wherein the first receiving part (6) and the second sliding part (4) comprise a respective connection interface for connecting elements of the medical appliance.

## Revendications

1. Dispositif (2) destiné à déterminer une profondeur de pénétration d'une partie coulissante (4) en forme de tube ou de tige, en particulier d'une partie porte-aiguille d'aspiration médicale dans une partie de réception (6), comportant une pièce de préhension (8) qui entoure la partie coulissante (4) et est formée pour coulisser le long d'un axe longitudinal (L) de la pièce coulissante (4) par rapport à la partie coulissante (4), et la pièce de préhension (8) comprenant :
- un élément de réglage (10) qui est formé pour tourner transversalement à l'axe longitudinal (L) de la partie coulissante (4), et
- au moins un élément oscillant (12) qui est agencé entre l'élément de réglage (10) et la partie coulissante (4),
- l'élément de réglage (10) étant formé pour faire pivoter l'élément oscillant (12) respectif dans une pose d'arrêt par rapport à la partie coulissante (4), dans laquelle le coulissement de la pièce de préhension (8) le long de l'axe longitudinal (L) est bloqué, par rotation dans une direction de blocage (B) transversalement à l'axe longitudinal (L) dans une position de blocage, et
- l'élément de réglage (10) étant formé pour faire pivoter l'élément oscillant (12) respectif dans une pose de libération par rapport à la partie coulissante (4), dans laquelle le coulissement de la pièce de préhension (8) le long de l'axe longitudinal (L) est libéré, par rotation dans une direction de réglage (V) différente de la direction de blocage (B) transversalement à l'axe longitudinal (L) dans une position de réglage différente de la position de blocage,
**caractérisé par**
- au moins un dispositif de fixation (16) ayant un premier élément de fixation (16a) pouvant tourner par rapport à la partie coulissante (4) transversalement à l'axe longitudinal (L) au moyen de l'élément de réglage (10), et un second élément de fixation (16b) fixe par rapport à la partie coulissante (4) à l'encontre de la rotation transversalement à l'axe longitudinal (L),
- les premier et second éléments de fixation (16a, 16b) étant formés de manière à exercer une force de retenue (H) dans la position de blocage afin de fixer l'élément de réglage (10) à l'encontre de la rotation transversalement à l'axe longitudinal (L),
- le premier élément de fixation (16a) étant entouré par l'élément de réglage (10) et la force de retenue (H) devant être surmontée pour libérer la fixation.

2. Dispositif selon la revendication 1, dans lequel le premier élément de fixation (16a) forme un corps de blocage et le second élément de fixation (16b) forme un évidement destiné à recevoir le corps de blocage, le corps de blocage étant formé pour être déplacé dans une pose de fixation, dans laquelle la force de retenue (H) est maximale, au moyen d'une manipulation d'un opérateur dans la position de blocage de l'élément de réglage (10).

3. Dispositif selon la revendication 1, dans lequel l'élément de réglage (10) est formé pour déplacer le premier élément de fixation (16a) dans une pose de fixation par rapport au second élément de fixation (16b), dans laquelle la force de retenue (H) est maximale, en tournant transversalement à l'axe longitudinal (L) dans la position de blocage.

4. Dispositif selon la revendication 3, dans lequel le premier élément de fixation (16a) et le second élément de fixation (16b) comprennent un matériau magnétique et forment un champ magnétique de polarité opposée.

5. Dispositif selon la revendication 3, dans lequel le premier élément de fixation (16a) est réalisé sous la forme d'un palier à roulement avec au moins un corps de roulement, et le second élément de fixation (16b) forme un évidement destiné à recevoir le corps de roulement dans la pose de fixation.

6. Dispositif selon l'une des revendications précédentes, dans lequel l'élément de réglage (10) et l'élément oscillant (12) respectif forment une structure de guidage (15) qui définit un trajet de guidage pour au moins faire tourner l'élément de réglage (10) transversalement à l'axe longitudinal (L) par rapport à l'élément oscillant (12) respectif, l'élément de réglage (10) étant réglable dans la position respective le long du trajet de guidage.

7. Dispositif selon la revendication 6, dans lequel la structure de guidage (15) comprend une portion de trajet de guidage associée à la position de blocage, qui s'étend le long de l'axe longitudinal (L), et l'élément de réglage (10) peut coulisser pour libérer la fixation le long de la portion de trajet de guidage par rapport à l'élément oscillant (12) respectif.

8. Dispositif selon l'une des revendications 3 à 7, dans lequel l'élément de réglage (10) est formé pour occuper une position de déblocage différente de la position de blocage et de la position de réglage en tournant à partir de la position de blocage dans la direction de blocage (8) afin de libérer la fixation, et en déplaçant ainsi les premier et second éléments de fixation (16a, 16b) dans une pose de déblocage, dans laquelle la force de retenue (H) est réduite par rapport à la pose de fixation, l'élément oscillant (12) respectif maintenant la pose d'arrêt dans la position de déblocage de l'élément de réglage (10).

9. Dispositif selon la revendication 8, dans lequel la pièce de préhension (8) comprend un dispositif de déblocage (18) qui est agencé entre la partie coulissante (4) et l'élément de réglage (10), et qui est formé pour maintenir les éléments de fixation (16a, 16b) respectifs dans la pose de déblocage en faisant tourner l'élément de réglage (10) transversalement à l'axe longitudinal.

10. Dispositif selon la revendication 9, dans lequel le dispositif de déblocage (18) comprend un premier élément de déblocage (20) qui est agencé de manière fixe par rapport à la partie coulissante (4) à l'encontre de la rotation transversalement à l'axe longitudinal, et l'élément de réglage (10) et le premier élément de déblocage (20) forment une structure d'espacement(11), la structure d'espacement(11) étant formée pour maintenir l'élément de réglage (10) et le premier élément de déblocage (20) à une distance prédéterminée l'un par rapport à l'autre le long de l'axe longitudinal (L) en faisant tourner l'élément de réglage (10) transversalement à l'axe longitudinal (L), et en réalisant ainsi la pose de déblocage des éléments de fixation (16a, 16b), et la structure d'espacement(11) comprenant un mécanisme d'assemblage au moyen duquel le premier élément de déblocage (20) et l'élément de réglage (10) sont assemblés par complémentarité de formes dans la position de blocage et la distance prédéterminée étant ainsi réduite.

11. Dispositif selon la revendication 10, dans lequel le dispositif de déblocage (18) comprend un élément de signalisation (24) qui fait saillie au moins dans certaines zones le long de l'axe longitudinal (L) au-delà de l'élément de réglage (10) afin de signaler le blocage et la fixation en faisant coulisser le premier élément de déblocage (20) uniquement dans la position de blocage.

12. Dispositif selon la revendication 11, dans lequel le dispositif de déblocage (18) comprend un second élément de déblocage (22) qui est agencé de manière fixe par rapport à l'élément de réglage (10) à l'encontre du coulissement le long de l'axe longitudinal (L), les premier et second éléments de déblocage (21, 22) formant une structure de coulissement (21), la structure de coulissement (21) étant formée pour déplacer l'élément de réglage (10) et le premier élément de déblocage (20) l'un par rapport à l'autre le long de l'axe longitudinal (L) à une distance prédéterminée l'un de l'autre en faisant tourner l'élément de réglage (10) transversalement à l'axe longitudinal (L) hors de la position de blocage dans la position de déblocage.

13. Dispositif selon la revendication 12, dans lequel l'élément de réglage (10) et le second élément de déblocage (22) forment une structure de couplage (13) pour transporter conjointement le second élément de déblocage (22) au moins dans certaines zones en faisant tourner l'élément de réglage (10) transversalement à l'axe longitudinal (L), la structure de couplage (13) étant formée pour décaler la structure d'espacement(11) et la structure de coulissement (21) transversalement à l'axe longitudinal (L) en faisant coulisser l'élément de réglage (10) de la position de déblocage à la position de réglage, et en maintenant ainsi la distance prédéterminée lors de la rotation dans la direction de réglage (V).

14. Pièce à main médicale pour un appareil médical, comportant un premier et un second dispositif (2) selon l'une des revendications précédentes, dans laquelle le premier dispositif (2) est formé pour déterminer la profondeur de pénétration d'une première partie coulissante en forme de tube (4) dans une première partie de réception (6), et dans laquelle le second dispositif (2) est formé pour définir la profondeur de pénétration d'une seconde partie coulissante en forme de tube (4) dans une seconde partie de réception (6), et le premier dispositif (2) et le second dispositif (2) sont agencés l'un derrière l'autre, de telle sorte que la première partie coulissante (4) du premier dispositif (2) est formée au moins en partie comme une seconde partie de réception (6) pour la seconde partie coulissante (4) du second dispositif (2), la première partie de réception (6) et la seconde partie coulissante (4) comportant une interface d'assemblage respective pour assembler des éléments de l'appareil médical.
